# EUROPEAN PATENT SPECIFICATION

(11) **EP 1 416 961 B1**
(45) Date of publication and mention of the grant of the patent: **17.05.2017**
(21) Application number: 02748438.5
(22) Date of filing: 12.07.2002
(51) Int. Cl.: A61K 39/395, A61K 38/17, A61K 48/00, A61K 31/4174, A61P 43/00

(54) **COMPOSITION FOR USE IN THE TREATMENT OF DISEASE**
ZUSAMMENSETZUNG UND VERFAHREN ZUR BEHANDLUNG VON KRANKHEITEN
COMPOSITION ET PROCEDE POUR LE TRAITEMENT D'UNE MALADIE

(30) Priority: 13.07.2001 AU PR638101
(43) Date of publication of application: 12.05.2004
(60) Divisional application: 08007413.1
(73) Proprietor: Paranta Biosciences Limited, Southbank VIC 3006 (AU)
(72) Inventor: PHILLIPS, David, Clayton, VIC 3168 (AU); DE KRETSER, David, Clayton, VIC 3168 (AU); SIEVERT, William, Clayton, VIC 3168 (AU); PATELLA, Shane, Clayton, VIC 3800 (AU); SMOLICH, Joseph, Parkville, VIC 3052 (AU); MCGAW, David, Windsor, VIC 3181 (AU); FENNESSY, Paul, Clayton, VIC 3800 (AU)
(74) Representative: Brasnett, Adrian Hugh
(86) International application number: PCT/AU2002/000945
(87) International publication number: WO 2003/006057

(56) References cited:
- WO-A-96/11259
- WO-A-99/10364
- OHGA EIJIRO ET AL.: 'Effects of activin A on proliferation and differentiation of human lung fibroblasts' BIOCHEMICAL AND BIOPHYSICAL RESEARCH COMMUNICATIONS vol. 228, no. 2, 1996, pages 391 - 396, XP002994067
- MATSUSE TAKESHI ET AL.: 'Expression of immunoreactive and bioactive activin A protein in adult murine lung after bleomycin treatment' AMERICAN JOURNAL OF RESPIRATORY CELL AND MOLECULAR BIOLOGY vol. 13, no. 1, July 1995, pages 17 - 24, XP008054587

## Description

### Technical Field

The present invention relates to compositions for use in the treatment of disease associated with fibrosis in a vertebrate via the administration of a therapeutically effective amount of follistatin.

### Background Art

A number of serious diseases of mammals, including humans, are associated with fibrosis. Such diseases include cirrhosis of the liver, pulmonary fibroses, and inflammatory bowel disease such as Crohn's disease.

Cirrhosis of the liver is a progressive disease of the liver characterised by diffuse damage to hepatic parenchymal cells with nodular regeneration, fibrosis and disturbance of normal architecture. It is associated with failure of hepatic cell function and interference with blood flow and can lead to total hepatic failure and hepatocellular carcinoma (HCC). There are a number of agents that cause hepatocellular injury including alcohol, the hepatitis viruses, various drugs and iron overload (Haemochromatosis) amongst others. Exposure to these agents promotes a cascade of events that, given repeated exposure, can result in the development of chronic disease including progressive fibrosis and cirrhosis.

Interstitial lung disease (ILD) is a term that includes a variety of chronic lung disorders. ILD is also referred to as interstitial pulmonary fibrosis or pulmonary fibrosis. The lung is usually damaged in some way, resulting in inflammation in the walls of the air sacs (alveolitis), in the walls of the bronchioles (bronchiolitis) or in the capillaries (vasculitis). Scarring (or fibrosis) then begins and the lung loses its elasticity. Fibrosis results in permanent loss of the lung tissue's ability to transport oxygen.

There is also a group of chronic lung diseases called idiopathic pulmonary fibrosis (IPF) which are of an unknown origin.

Inflammatory bowel disease (IBD) is a group of chronic disorders that cause inflammation or ulceration in the small and large intestines. Most often IBD is classified as ulcerative colitis or Crohn's Disease but may be referred to as colitis, enteritis, ileitis, and proctitis. Ulcerative colitis causes ulceration and inflammation of the inner lining of the colon and rectum, while Crohn's Disease is an inflammation that extends into the deeper layers of the intestinal wall. The intestine wall is thickened and at first is pliable, but as fibrosis occurs the wall becomes stiff with lumenal narrowing and occasional stenosis. Crohn's Disease also may affect other parts of the digestive tract, including the mouth, oesophagus, stomach, and small intestine.

WO94/09809 relates to a method of preventing or treating liver disease using an antagonist to activin. WO96/11259 relates TGF-β1, activin receptors 1 and 3, and their use. WO99/10364 relates to isolation and characterisation of follistatin-3 and its encoding nucleotide sequence. Ohga et al. (Biochem Biophys Res Commun. 1996, 228(2):391-6) relates to the effects of activin A on proliferation and differentiation of human lung fibroblasts. Matsuse et al. (Am J Respir Cell Mol Biol. 1995, 13(1):17-24) relates to expression of immunoreactive and bioactive activin A protein in adult murine lung after bleomycin treatment.

New antifibrotic treatments to prevent development of, or cure fibrosis-associated conditions, such as those identified above, are needed as these conditions are major causes of death and/or loss of quality of life and increased burden on medical systems.

It is now disclosed herein that activin antagonists may be used as agents for treatment and/or prevention of fibrosis, and associated diseases.

Accordingly, the present invention describes a role for activin antagonists that will be useful for the treatment and/or prevention of disease associated with fibrosis.

Therefore, the present invention provides a useful therapeutic in the treatment of disease associated with fibrosis, in the form of activin antagonist follistatin.

### Disclosure of the Invention

The present invention relates to the finding that follistatin is useful therapeutics for the treatment and/or prophylaxis of disease associated with fibrosis. Typically, fibrosis may involve abnormally elevated expression of the hormone activin A. The results disclosed herein support the position that administration of an activin antagonist, particularly follistatin, inhibits the hyperproliferation associated with the hormone activin.

### Therapeutic/Pharmaceutical Compositions for Treatment and/or Prophylaxis of Disease

Herein disclosed is a pharmaceutical composition for the treatment and/or prophylaxis of disease associated with fibrosis in.a vertebrate, said composition comprising at least one activin antagonist, and optionally a pharmaceutically acceptable carrier, adjuvant and/or diluent.

The invention provides follistatin for use in a method of treatment or prophylaxis of disease associated with fibrosis in a vertebrate in need of said treatment or prophylaxis, wherein said disease is selected from: hyperproliferative or inflammatory fibrotic diseases; interstitial lung diseases; inflammatory bowel disease, ulcerative colitis and Crohn's Disease; tubular necrosis in the kidney; and liver fibrosis.

In another aspect, the invention provides a pharmaceutical composition for the treatment or prophylaxis of disease associated with fibrosis in a vertebrate, characterised in that said composition comprises follistatin and optionally a pharmaceutically acceptable carrier, adjuvant and/or diluent, wherein said composition provides a dosage of from 0.0001 to 10 mg follistatin per kg body weight per 24 hours.

The invention further provides process for preparing the pharmaceutical composition of the above aspect, characterised in that said process comprises homogeneously mixing follistatin with a pharmaceutically acceptable carrier, adjuvant and/or diluent.

Typically, the vertebrate is selected from the group consisting of human, non-human primate, mice, cattle, sheep, goats, horses, rabbits, birds, cats and dogs. More typically, the vertebrate is human, non-human primate or mouse. Even more typically, the vertebrate is human.

### 2. Treatment of Disease using Follistatin

Also described herein is a method for the treatment of disease associated with fibrosis in a vertebrate in need of said treatment, wherein said method comprises administering to said vertebrate, a therapeutically effective amount of at least one activin antagonist.

Also described herein is at least one activin antagonist when used in the treatment of disease associated with fibrosis in a vertebrate in need of said treatment.

Also described herein is the use of at least one activin antagonist for the preparation of a medicament for the treatment of disease associated with fibrosis in a vertebrate in need of said treatment.

Typically, for the purposes described above, the activin antagonist is typically follistatin, or a fragment(s) or analogue thereof, as herein described.

Also described herein is a method for the treatment of disease associated with fibrosis in a vertebrate in need of said treatment, wherein said method comprises administering to said vertebrate, a therapeutically effective amount of the pharmaceutical composition as defined in the first embodiment of the invention.

Also described herein is a pharmaceutical composition as defined in the first embodiment of the invention, when used in the treatment of disease associated with fibrosis in a vertebrate in need of said treatment.

Also described herein is the use of the pharmaceutical composition as defined in the first embodiment of the invention for the preparation of a medicament for the treatment of disease associated with fibrosis in a vertebrate in need of said treatment.

According to one preferred aspect, the disease associated with fibrosis is a hyperproliferative or inflammatory fibrotic diseases.

According to another preferred aspect, the disease associated with fibrosis is a pulmonary fibrosis, such as idiopathic pulmonary fibrosis or an interstitial lung disease.

According to another preferred aspect, the disease associated with fibrosis is an inflammatory bowel disease, or a related condition such as ulcerative colitis or Crohn's Disease.

According to another preferred aspect, the disease associated with fibrosis is liver fibrosis and cirrhosis.

Typically, the treatment of disease associated with fibrosis through the administration of a therapeutically effective amount of an activin antagonist is undertaken in conjunction with other treatments of disease. For example, these other treatments may include: surgery, radiation treatment, or chemotherapy. For instance, the chemotherapy may involve the administration of anti-fibrotic, anti-thrombotic or anti-inflammatory drugs.

Typically, for the purposes of any one of the above described embodiments of the invention, one skilled in the art would be able, by routine experimentation, to determine what an effective, non-toxic amount of an activin antagonist would be for the purpose of treating the disease.

For the purposes of the embodiments of the invention described above, the activin antagonist is follistatin and more typically the follistatin is a single chain protein comprising between 288 and 315 amino acids with a molecular weight of between about 30,000 and 60,000 Daltons as estimated by SDS-PAGE in the absence of reducing agents, derived from follicular fluid and able to inhibit the secretion of follicle-stimulating hormone (FSH). More typically follistatin is a single chain protein classified as NCBI (National Center for Biotechnology Information) protein XP_003891, AAH04107. Even more typically, follistatin is as described in Australian Patent No 610858. Still more typically, follistatin is as described in Australian Patent No. 620346 or United States Patent No. US5,470,826 or European Patent No. EP 0299 050.

Typically, the follistatin present in the pharmaceutical composition may also exist in a form selected from the group consisting of: follistatin/chelate, follistatin/drug, follistatin/prodrug, follistatin/toxin and follistatin/detector group and follistatin/imaging marker.

### 3. Diagnosis of Disease.

Also described herein is a method for screening for a disease associated with fibrosis in a vertebrate comprising:
(a) contacting a sample from the vertebrate with an antibody (or fragment thereof) raised against an activin polypeptide (or fragment or analogue thereof);
(b) detecting the presence of the antibody (or fragment thereof) bound to the activin polypeptide; and
(c) comparing the amount of bound antibody to the amount bound in a reference sample, and diagnosing a disease associated with fibrosis in said vertebrate, wherein a change in the amount of bound antibody in the sample compared to the reference sample is indicative of disease.

Also described herein is a method for screening for a disease associated with fibrosis in a vertebrate comprising:
(a) contacting a sample from the vertebrate with an antibody (or fragment thereof) raised against a follistatin polypeptide (or fragment or analogue thereof);
(b) detecting the presence of the antibody (or fragment thereof) bound to the follistatin polypeptide; and
(c) comparing the amount of bound antibody to the amount bound in a reference sample, and diagnosing a disease associated with fibrosis in said vertebrate, wherein a change in the amount of bound antibody in the sample compared to the reference sample is indicative of disease.

Also described herein is a method for screening for a disease associated with fibrosis in a vertebrate comprising:
(a) contacting a first aliquot of a sample from the vertebrate with an antibody (or fragment thereof) raised against an activin polypeptide (or fragment or analogue thereof);
(b) detecting the presence of the antibody (or fragment thereof) bound to the activin polypeptide; and
(c) contacting a second aliquot of a sample from the vertebrate with an antibody (or fragment thereof) raised against a follistatin polypeptide (or fragment or analogue thereof);
(d) detecting the presence of the antibody (or fragment thereof) bound to the follistatin polypeptide; and
(e) comparing the amount of activin-bound antibody to the amount of follistatin-bound antibody, and comparing the relative difference to that found in a reference sample, and diagnosing a disease associated with fibrosis in said vertebrate, wherein a change in the relative ratio of activin- and follistatin-bound antibody in the sample compared to the reference sample is indicative of disease.

Typically, for the purposes of the screening methods described above, the reference sample is obtained from a vertebrate not suffering from a disease associated with fibrosis.

Typically, for the purposes of the screening methods, the sample within which the method of screening is performed is a plasma or tissue sample, and involves standard histological and immunohistochemical techniques.

Also described herein is a diagnostic kit for the detection of a disease associated with fibrosis in a vertebrate, said kit comprising at least an antibody (or fragment thereof) raised against activin (or fragment thereof), together with a diagnostically acceptable carrier and/or diluent.

Also described herein is a diagnostic kit for the detection of disease associated with fibrosis in a vertebrate, said kit comprising at least an antibody (or fragment thereof) raised against follistatin (or fragment thereof), together with a diagnostically acceptable carrier and/or diluent.

Typically, the kits above-described may comprise the following containers:
(a) a first container containing at least the antibody (or fragment thereof), and;
(b) a second container containing a conjugate comprising a binding partner of the antibody (or fragment thereof), together with a detectable label.

Also described herein is a diagnostic kit for the detection of disease associated with fibrosis in a vertebrate, said kit comprising at least: an antibody (or fragment thereof) raised against follistatin (or fragment thereof), together with a diagnostically acceptable carrier and/or diluent; and an antibody (or fragment thereof) raised against activin (or fragment thereof), together with a diagnostically acceptable carrier and/or diluent.

Typically, the above kit may comprise the following containers:
(a) a first container containing at least an activin antibody (or fragment thereof), and;
(b) a second container containing at least a follistatin antibody (or fragment thereof);
(c) a third container containing a conjugate comprising a binding partner of the activin antibody (or fragment thereof), together with a detectable label, and
(d) a fourth container containing a conjugate comprising a binding partner of the follistatin antibody (or fragment thereof), together with a detectable label.

More typically, the above-described kits may further comprise one or more other containers, containing other components, such as wash reagents, and other reagents capable of detecting the presence of bound antibodies. Even more typically, the detection reagents may include labelled (secondary) antibodies or, where the antibody (or fragment thereof) raised against activin and/or follistatin (or fragment thereof) is itself labelled, the compartments may comprise antibody binding reagents capable of reacting with the labelled antibody (or fragment thereof) of the present invention.

### 4. Gene Therapy

In another aspect, the invention provides a nucleic acid molecule encoding for follistatin or a vector comprising said nucleic acid molecule for use in a method of treatment or prophylaxis of disease associated with fibrosis in a vertebrate.

Typically, for the purposes of gene therapy, the nucleic acid molecule or vector is inserted using methods selected from the group consisting of: microinjection, CaPO₄ precipitation, electroporation, lipofection/liposome fusion, particle bombardment and coupling the nucleic acid to chemically modified proteins.

Typically the nucleic acid molecule or vector is inserted into the nucleus of a host cell.

Typically, an expression vector containing the nucleic acid molecule is inserted into cells, the cells are grown *in vitro* and then infused in large numbers into patients. More typically, expression vectors derived from viruses such as adenovirus, adeno-associated virus, vaccinia virus, herpes viruses, several RNA viruses, retroviruses, or bovine papilloma virus, may be used for delivery of the nucleic acid into the targeted cell(s). More typically, the targeted cell(s) comprise fibroblast lineages eg hepatic stellate cells, or smooth muscle cells, lung fibroblasts, myofibroblasts, kidney cells.

### Brief Description of the Drawings

Figure 1 shows expression of activin A (Fig. 1A) and follistatin (Fig. 1B) in normal liver section by immunohistochemistry.
Figure 2 shows activin expression in cirrhotic liver section by immunohistochemistry.
Figure 3 shows confocal microscopy of rat liver sections showing activin A and alpha-smooth muscle actin expression.
Figure 4 shows follistatin expression in fibrotic animal liver section by immunohistochemistry
Figure 5 shows expression of both activin A and follistatin mRNA in whole liver extracts during model of CCl₄ rat liver injury.
Figure 6 shows real time PCR analysis on freshly isolated hepatic stellate cells (HSCs) as they transdifferentiated *in vitro* to determine the expression pattern of activin A and follistatin in relation to other key markers of HSC proliferation and extracellular matrix (ECM) production.
Figure 7 shows secretion of activin A protein by primary cultures of HSCs as they transformed to the activated phenotype.
Figure 8 shows the decrease in proliferation of MPC-11 cells (counts per minute, ³H thymidine incorporation) after addition of activin A-containing supernatants from HSC cultures.
Figure 9 shows the effect of various exogenous mediators on freshly isolated HSC proliferation (% proliferation as compared to control vs concentration of added exogenous mediator): Fig. 9A shows results for activin as exogenous mediator; Fig. 9B shows results for transforming growth factor β (TGF) as exogenous mediator; Fig. 9C shows results for follistatin as exogenous mediator; Fig. 9D shows results for platelet derived growth factor (PDGF) as exogenous mediator.
Figure 10 shows the effect of various exogenous mediators on activated/transdifferentiated HSC proliferation (% proliferation as compared to control vs concentration of added exogenous mediator): Fig. 10A shows results for activin as exogenous mediator; Fig. 10B shows results for transforming growth factor β (TGF) as exogenous mediator; Fig. 10C shows results for follistatin as exogenous mediator; Fig. 10D shows results for platelet derived growth factor (PDGF) as exogenous mediator.
Figure 11 shows the effect of exposure to varying quantities of activin A, follistatin and TGFβ to cultures of activated HSC's on cell viability (assessed by flow cytometry for the expression of annexin V, an early marker of cellular apoptosis).
Figure 12 shows change in body weight of control rats compared to rats exposed to CCl₄ for 4 weeks and then co-injected with 1 µg of follistatin 3 times a week for the first 4 weeks and then sacrificed. Control animals received CCl₄ for the same length of time.
Figure 13 shows remnant liver weight for the same experimental conditions described for Figure 12.
Figure 14 shows intrahepatic hydroxyproline content for the same experimental conditions described for Figure 12.
Figure 15 shows change in body weight of control rats compared to rats exposed to CCl₄ for 8 weeks and then co-injected with 1 µg of follistatin 3 times a week from weeks 8-12 and then sacrificed. Control animals received CCl₄ for the same length of time.
Figure 16 shows remnant liver weight for the same experimental conditions described for Figure 15.
Figure 17 shows intrahepatic hydroxyproline content for the same experimental conditions described for Figure 15.
Figure 18 shows serum activin A in human subjects with chronic viral hepatitis and normal controls.
Figure 19 shows serum follistatin in human subjects with chronic viral hepatitis and normal controls.
Figure 20 shows correlation of serum activin A in patients with hepatitis B (HBV) with serum alanine aminotransferase (ALT, a marker for hepatocyte injury and intrahepatic injury).
Figure 21 shows Correlation of serum activin A in patients with HBV and viral replication (assayed as serum HBV, pg/mL).
Figure 22 shows negative correlation of serum follistatin in patients with HBV and viral replication (assayed as serum HBV, pg/mL).

### Definitions

The term "activin antagonist" encompasses molecules that inhibit activin activity. The term includes molecules that bind to activin and molecules that antagonise activin by binding to the activin receptor (type I or II) to block downstream signalling. For example, molecules that inhibit activin activity by binding to activin include follistatin, follistatin-related protein (Genbank accession number NP_005851), and alpha-2 macroglobulin, and molecules that antagonise activin by binding to the activin receptor (type I or II) to block downstream signalling include inhibin. "Activin antagonists" may also include: molecules that interfere with any of the other downstream components of activin signal transduction pathway, such as the inhibitory Smad signalling molecules, Smad6 and 7; dominant negative mutants of the activin receptor (eg BAMBI) which if expressed in a cell will interfere with that cell's activin signal transduction pathway; molecules that specifically inhibit TGFβ/activin type I receptors such as triarylimidazole analogues as are described in Callahan, J.F., et al (2002), "Identification of novel Inhibitors of the Transforming Growth Factor β-1 (TGF-β1) Type I Receptor (ALK5)", J. Med. Chem 45: 999-1001.

The term "nucleic acid" encompasses single or double-stranded deoxyribonucleotide (DNA) and/or ribonucleotide (RNA) nucleic acid, including all known analogues of natural nucleotides.

The term "polynucleotide" encompasses single or double-stranded deoxyribopolynucleotide and/or ribopolynucleotide, including all known analogues of natural nucleotides. It also includes within its scope the relevant sequence as specified, together with the sequence complementary thereto.

As used herein the term "polypeptide" refers to a polymer made up of a plurality of amino acids linked together by peptide bonds.

The term "antibody" refers to an immunoglobulin molecule able to bind to a specific epitope on an antigen, and which may be comprised of a polyclonal mixture, or be monoclonal in nature. Antibodies may be entire immunoglobulins derived from natural sources, or from recombinant sources. An antibody according to the present invention may exist in a variety of forms including, for example, whole antibody, an antibody fragment, or another immunologically active fragment thereof, such as a complementarity determining region. Similarly, the antibody may be an antibody fragment having functional antigen-binding domains, that is, heavy and light chain variable domains. The antibody fragment may also exist in a form selected from the group consisting of: Fv, F_{ab}, F(ab)₂, scFv (single chain Fv), dAb (single domain antibody), bi-specific antibodies, diabodies and triabodies.

The term "antisense" pertaining to nucleic acid molecules, as referred to herein, means an artificial oligo- or polynucleotide molecule which is complementary to a target polypeptide encoding nucleotide sequence. The antisense nucleic acid molecule may be transcribed in a cell, and is capable of hybridising to the polypeptide-encoding mRNA produced in the cell. On the basis that the reaction occurs under conditions allowing the complementary antisense nucleotide sequence to hybridise to the polypeptide mRNA, the amount of polypeptide translated is thus altered, that is, reduced or eliminated.

A "therapeutically effective amount", as referred to herein, includes a sufficient, but non-toxic amount of a compound or composition of the invention to provide the desired therapeutic effect. The "effective amount" will vary from subject to subject depending on one or more of a number of factors amongst, for example, the particular agent being administered, the severity of the condition being treated, the species being treated, the age and general condition of the subject and the mode of administration. For any given case, an appropriate "effective amount" may be determined by one of ordinary skill in the art using only routine experimentation. Typically, "therapeutically effective amount" refers to an amount sufficient to result in one or more or the following: recession/reduction in the extent of the disease, inhibition of disease growth or progression, cessation of disease growth, relief of disease-imposed discomfort, or prolongation of life of the vertebrate having the disease.

The term "isolated" indicates that the material in question has been removed from its naturally existing environment, and associated impurities reduced or eliminated. Essentially, the 'isolated' material is enriched with respect to other materials extracted from the same source (ie., on a molar basis it is more abundant than any other of the individual species extracted from a given source), and preferably a substantially purified fraction is a composition wherein the 'isolated' material comprises at least about 30 percent (on a molar basis) of all macromolecular species present. Generally, a substantially pure composition of the material will comprise more than about 80 to 90 percent of the total of macromolecular species present in the composition. Most preferably, the 'isolated' material is purified to essential homogeneity (contaminant species cannot be detected in the composition by conventional detection methods) wherein the composition consists essentially of the subject macromolecular species.

"Conservative amino acid substitutions" refer to the interchangeability of residues having similar side chains. For example, a group of amino acids having aliphatic side chains includes glycine, alanine, valine, leucine, and isoleucine; a group of amino acids having aliphatic-hydroxyl side chains includes serine and threonine; a group of amino acids having amide-containing side chains includes asparagine and glutamine; a group of amino acids having aromatic side chains includes phenylalanine, tyrosine, and tryptophan; a group of amino acids having basic side chains includes lysine, arginine, and histidine; and a group of amino acids having sulfur-containing side chains includes cysteine and methionine. Typically, conservative amino acids substitution groups are: valine-leucine-isoleucine, phenylalanine-tyrosine, lysine-arginine, alanine-valine, and asparagine-glutamine.

The term "fragment" of a compound, with reference to polypeptides, is a compound having qualitative biological activity in common with for example, a full-length polypeptide from which it is derivable.

With reference to nucleic acids, nucleotides and/or polynucleotides, the term "fragment" relates to compounds including, for example: portions of a target nucleic acid sequence which encode a product having qualitative biological activity in common with for example, a full-length polypeptide derivable from the full length nucleic acid sequence; or fragments of a target nucleic acid sequence which are suitable as specific probes or PCR primers for the detection and/or amplification of the target nucleic acid sequence, or a functional product-encoding portion thereof.

The term "analogue" as used herein with reference to a nucleic acid sequence means a sequence which is a derivative of a target nucleic acid sequence, the derivative comprising addition, deletion, or substitution (including conservative amino acid substitutions) of one or more bases and wherein the encoded polypeptide retains substantially the same function as the polypeptide encoded by the target nucleic acid molecule. Similarly, the term "analogue" as used herein refers to a derivative of a target polypeptide comprising addition, deletion, or substitution of one or more amino acids, the analogue retaining, however, substantially the same function as the target polypeptide.

The term "expression cassette" refers to a nucleic acid construct comprising the necessary nucleic acid elements (promoters, enhancers, the nucleic acid to be transcribed, etc) which permit the transcription of the particular nucleic acid in a host cell. The expression construct can be incorporated into a vector, host chromosome etc.

The term "promoter" refers to nucleic acid sequences that influence and/or promote initiation of transcription.

The term "operably linked" refers to the situation wherein, for example, a nucleic acid is placed into a functional relationship with another nucleic acid sequence. For example, a promoter operably linked to a heterologous DNA, which encodes a protein, promotes the production of functional mRNA corresponding to the heterologous DNA.

As used herein "gene transfer" means the process of introducing foreign genetic material into a cell, and is commonly performed to enable the expression of a particular product encoded by the gene. The product may include a protein, polypeptide, anti-sense DNA or RNA, or enzymatically active RNA. Gene transfer can be performed in cultured cells or by direct administration into animals, and generally involves the process of contacting a target cell with a desired nucleic acid by non-specific or receptor mediated interactions, uptake of nucleic acid into the cell through the membrane or by endocytosis, and release of nucleic acid into the cytoplasm from the plasma membrane or endosome. For successful expression, movement of the nucleic acid into the nucleus of the cell and binding to appropriate nuclear factors for transcription may also be required where the nucleic acid of interest is part of an expression construct.

By the term "gene therapy" includes gene transfer and antisense biotechnological techniques, as referred to above. "Gene therapy" specifically refers to either gene transfer to express a therapeutic product from a cell *in vivo* or *in vitro,* or to antisense techniques whereby oligo- or polynucleotide sequences complementary for a target polynucleotide-encoding sequence(s) is inserted into and expressed in cells *in vivo* or *in vitro* so as to impede production of the target polypeptide. Gene transfer can be performed: *ex vivo* on cells which are then transplanted into a patient; by direct administration of the nucleic acid or nucleic acid-protein complex into the patient; or by transfer of modified cells into a patient. Antisense techniques can be performed *in vivo* using appropriate transfection vectors to deliver expression vectors comprising the antisense-encoding sequence(s) to target cells.

As used herein the term "treatment", refers to any and all uses which remedy a disease state or symptoms, or otherwise prevent, hinder, retard, or reverse the progression of disease or other undesirable symptoms in any way whatsoever.

In the context of this specification, the term "comprising" means "including principally, but not necessarily solely". Variations of the word "comprising", such as "comprise" and "comprises", have correspondingly varied meanings.

### Best Mode of Performing the Invention

The present invention describes the use of activin antagonists, as exemplified by follistatin, for use in the treatment of a number of other disorders, a common feature of which is the association of these disorders with fibrosis. Follistatin is best known for its involvement in the suppression of follicle-stimulating hormone, with a consequential role in the treatment of fertility disorders, but has now been found to be an effective activin antagonist capable of inhibiting hyperproliferation of cells associated with fibrotic diseases.

Methods of making activin antagonists, where these are proteins such as follistatin can employ conventional techniques of molecular biology, microbiology, recombinant DNA and immunology, all of which are within the skill of the art and fully explained in any one of a number of well known scientific publications, such as: "Molecular Cloning: A Laboratory Manual" Second Edition by Sambrook et al., Cold Spring Harbor Press, 1989. For example, the gene for follistatin may be isolated from cells or tissues that express follistatin by: isolating messenger RNA from the tissue or cells, using reverse transcriptase to generate the corresponding DNA sequence, and finally using the polymerase chain reaction (PCR) with the appropriate primers to amplify the DNA sequence coding for the active follistatin amino acid sequence. Also, a polynucleotide encoding a follistatin fragment may be cloned into an appropriate expression vector, and then expressed in a suitable procaryotic, viral or eucaryotic host. Expressed follistatin polypeptides can be purified according to standard procedures known in the art, including one or more of the following established procedures: protein precipitation, including ammonium sulfate, ethanol or polyethylene glycol precipitation and immuno-precipitation; chromatographic techniques using ionexchange, size exclusion, reverse-phase, hydrophobic interaction, affinity, or immunoaffinity technologies and carried out by, for example, column chromatography, HPLC, or FPLC; electrophoretic techniques such as gel electrophoresis and HPEC; and the like.

For instance, for producing recombinant follistatin or active fragment(s) thereof for use in the present invention, the relevant DNA sequences are inserted into a suitable expression system. Preferably, a recombinant molecule or vector is constructed in which the polynucleotide sequence encoding follistatin is operably linked to a heterologous expression control sequence enabling expression of the follistatin protein. A number of appropriate expression vectors are known in the art for mammalian (including human) protein expression, and employed using standard molecular biology techniques. Such vectors may be selected from among conventional vector types including insects, such as baculovirus expression, or yeast, fungal, bacterial or viral expression systems.

Suitable host cells or cell lines for transfection in such a method include mammalian cells, such as Human 293 cells, Chinese hamster ovary cells (CHO), the monkey COS-1 cell line or murine 3T3 cells. Similarly bacterial cells such as the various well-known strains of *E. coli* (e.g., HB101, MC1061), and various strains of *B. subtilis, Pseudomonas*, other bacilli and the like are useful as host cells for the present invention. Many strains of yeast cells known to those skilled in the art are also available as host cells for expression of the polypeptides of the present invention. Insect cells such as *Spodoptera frugipedera* (Sf9) cells may also be used.

The vectors containing the DNA segments of interest can be transferred into the host cell by any one of a number of well-known methods, depending on the type of cellular host. For example, calcium chloride transfection and electroporation are commonly utilised for procaryotic cells. Calcium phosphate treatment, electroporation, lipofection, biolistics or viral-based transfection may be used for other cellular hosts. Methods for transforming mammalian cells may also include the use of transfection, transformation, conjugation, polybrene, liposomes, electroporation, particle gun technology and microinjection (see, generally, Sambrook *et al.,* 1989).

Recombinant host cells are then advantageously grown in a selective medium, which inherently selects for the growth of those cells containing the introduced vector. The incubation conditions are ideally selected to optimise expression of the recombinant polypeptide.

Therefore, for use in the present invention recombinant activin antagonist(s) may be produced by transfecting a host cell with at least one expression vector containing a recombinant polynucleotide encoding the activin antagonist follistatin under the control of a transcriptional regulatory sequence. The transformed cell is then cultured under conditions that allow expression of the follistatin protein. The expressed protein may then be recovered from the cell or the culture medium, isolated, and optionally purified by appropriate means known to one of skill in the art. For example, the proteins may be isolated in soluble form following cell lysis, or may be extracted using known techniques, such as in guanidine chloride.

For example, microbial cells containing the exogenous follistatin gene may be cultured in large volume reactors, collected by centrifugation and then ruptured by, for example, high pressure homogenisation. The resulting cell lysate may be resuspended in an appropriate diluent/ buffer, and filtered to obtain an aqueous suspension of the follistatin protein. The recombinant protein can be administered in crude form, for example, by diluting in a 0.1M phosphate buffer (pH 7.4) to 50-500µg/ml concentration, and then passing through a sterile 0.22 micron filter.

Activin antagonists such as follistatin may also be synthesised by methods of solid phase chemistry well known to those of ordinary skill in the art. For example, follistatin fragments may be synthesised following the solid phase chemistry procedures of Steward and Young (Steward, J. M. & Young, J. D., Solid Phase Peptide Synthesis. (2nd Edn.) Pierce Chemical Co., Illinois, USA (1984). In general, such a synthesis method comprises the sequential addition of one or more amino acids or suitably protected amino acids to a growing peptide chain. Typically, functional group(s) other than one of either the amino or carboxyl group of the first amino acid is/are protected by a suitable protecting group. The protected amino acid is then either attached to an inert solid support or utilised in solution by adding the next amino acid in the sequence having the complementary (amino or carboxyl) group suitably protected and under conditions suitable for forming the amide linkage. The protecting group is then removed from this newly added amino acid residue and the next (protected) amino acid is added, and so forth. After all the desired amino acids have been linked, any remaining protecting groups, and if necessary any solid support, is removed sequentially or concurrently to produce the final polypeptide.

Amino acid changes in the activin antagonist, such as in the follistatin polypeptide or fragment thereof may be effected by techniques well known to those persons skilled in the relevant art. For example, amino acid changes may be effected by the addition, deletion or substitution of nucleotides (conservative and/or non-conservative), whilst maintaining the proper reading frame. Such modifications in the target polynucleotide may be produced by techniques including random mutagenesis, site-directed mutagenesis, oligonucleotide-mediated or polynucleotide-mediated mutagenesis, deletion of selected region(s) through the use of existing or engineered restriction enzyme sites, and the polymerase chain reaction.

The activin antagonist of the invention may alternatively be produced as a fusion protein. For instance, it may be desirable to produce follistatin fusion proteins, to enhance expression of the protein in a selected host cell, to improve purification, or for use in monitoring the presence of follistatin in tissues, cells or cell extracts. Suitable fusion partners are well known to those of skill in the art and include: β-galactosidase, glutathione-S-transferase, and poly-histidine.

The activin antagonist of the invention may typically also be an antibody raised against activin or an activin-receptor.

An antibody (or fragment thereof) may be raised against activin, activin receptors, or immunogenic portions thereof using the methods described below. For convenient production of adequate amounts of antibody(s), these may be manufactured by batch fermentation with serum free medium, and then purified via a multistep procedure incorporating chromatography and viral inactivation/removal steps. For example, the antibody may be first separated by Protein A affinity chromatography and then treated with solvent/detergent to inactivate any lipid enveloped viruses. Further purification, typically by size-exclusion, reverse-phase, anion and/or cation exchange chromatographies, may be used to remove residual undesired contaminants such as proteins, solvents/detergents and nucleic acids. The antibody(s) thus obtained may be further purified and formulated into 0.9% saline using gel filtration columns. The formulated bulk preparation may then be sterilised and viral filtered and dispensed.

These antibodies can include but are not limited to polyclonal, monoclonal, chimeric, single chain, Fab fragments, and an Fab expression library.

A monoclonal antibody refers to an antibody secreted by a single clone of antibody-producing cells and which is monospecific for a particular antigen or epitope. Therefore, a monoclonal antibody displays a single binding affinity for any antigen with which it immunoreacts.

Activin or activin receptor antibodies may be raised using methods well known to those skilled in the art. For instance, a monoclonal antibody, typically containing Fab portions, may be prepared using the hybridoma technology described in Antibodies-A Laboratory Manual, Harlow and Lane, eds., Cold Spring Harbor Laboratory, N.Y. (1988). Any technique that provides for the production of antibody molecules by continuous cell lines in culture may be used. Suitable techniques include the hybridoma technique originally developed by Kohler et al., Nature, 256:495-497 (1975), the trioma technique, the human B-cell hybridoma technique [Kozbor et al., Immunology Today, 4:72 (1983)], and the EBV-hybridoma technique to produce human monoclonal antibodies [Cole et al., in Monoclonal Antibodies and Cancer Therapy, pp. 77-96, Alan R. Liss, Inc., (1985)]. Immortal, antibody-producing cell lines can be created by techniques other than fusion, such as direct transformation of B lymphocytes with oncogenic DNA, or transfection with Epstein-Barr virus. See, e.g., M. Schreier et al., "Hybridoma Techniques" (1980); Hammerling et al., "Monoclonal Antibodies and T-cell Hybridomas" (1981); Kennett et al., "Monoclonal Antibodies" (1980).

There are also various procedures known in the art which may be used for the production of polyclonal antibodies to activin, activin receptors, or fragments thereof. For example, one or more host animals can be immunised by injection with the relevant polypeptide, or a derivative (e.g., fragment or fusion protein) thereof. Suitable hosts include, for example, rabbits, mice, rats, sheep, goats, etc.

The antibody (or fragment thereof) will have a binding affinity or avidity. Preferably, this binding affinity or avidity is greater than about 10⁵ M⁻¹, more preferably greater than about 10⁶ M⁻¹, more preferably still greater than about 10⁷ M⁻¹ and most preferably greater than about 10⁸ M⁻¹.

### 1. Treatment and/or Prophylaxis of Disease using Activin Antagonists

The administration of activin antagonists as described in the present invention is useful for treating fibrotic-dependent diseases in vertebrates, especially hyperproliferative or inflammatory fibrotic diseases; inflammatory bowel disease, and related conditions such as ulcerative colitis and Crohn's Disease; tubular necrosis in the kidney together with liver fibrosis.

Typically, the vertebrate is selected from the group consisting of human, non-human primate, mice, cattle, sheep, goats, horses, rabbits, birds, cats and dogs. More typically, the vertebrate is human, non-human primate or mouse. Even more typically, the vertebrate is human.

The therapeutically effective dose level for any particular patient will depend upon a variety of factors including: the disorder being treated and the severity of the disorder; activity of the activin antagonist employed; the composition employed; the age, body weight, general health, sex and diet of the patient; the time of administration; the route of administration; the rate of excretion of the activin antagonist; the duration of the treatment; drugs used in combination or coincidental with the activin antagonist together with other related factors well known in medicine. For example, it is well known in the art to begin doses of a therapeutic compound at levels lower than those expected to achieve a desired therapeutic effect, and to gradually increase the dosage, if necessary, until the desired effect is achieved.

Therefore, determination of an effective, non-toxic amount of an activin antagonist required to treat a disorder/ disease to which the antagonist is applicable can be readily determined by an appropriately skilled person by no more than routine experimentation. Typically, for follistatin, an effective dosage is expected to be in the range of about 0.00001 to about 100mg follistatin per kg body weight per 24 hours, preferably about 0.0001 to about 10mg follistatin per kg body weight per 24 hours, more preferably about 0.001 to about 1mg follistatin per kg body weight per 24 hours, even more preferably about 0.002 to about 0.5mg follistatin per kg body weight per 24 hours, even more preferably still about 0.005 to about 0.20mg follistatin per kg body weight per 24 hours. Further, if desired, the effective daily dose may be divided into multiple doses for purposes of administration.

Alternatively, an effective dosage of follistatin may be up to about 6,500mg/m² per 24 hours. Generally, an effective dosage is expected to be in the range of about 0.004 to about 400mg/m², preferably about 0.04 to about 40mg/m², more preferably about 0.08 to about 20mg/m², still more preferably about 0.2 to about 8mg/m².

Typically the treatment would be for the duration of the condition, and contact times would typically be for the duration of the condition.

Clearly the optimal quantity and spacing of individual dosages of a compound of the present invention will be determined by the nature and extent of-the condition being treated, the form, route and site of administration, and the nature of the particular vertebrate being treated, and these optimum conditions can be determined by routine procedures by skilled persons in the field.

Also disclosed herein are prodrugs of activin antagonists. Typically, these prodrugs are functional derivatives of follistatin which are readily converted *in* vivo. Typical procedures for the selection and preparation of prodrugs are established and described in available texts such as, for instance, H. Bundgaard (Ed), Design of Prodrugs, Elsevier, 1985.

When used in the treatment of disease, the activin antagonist may be administered alone. However, it is generally preferable that the activin antagonist be administered in conjunction with other chemotherapeutic treatments conventionally administered to patients for treating disease.

Pharmaceutical formulations of the present invention will typically be prepared by methods known to those of ordinary skill in the art and will therefore typically include excipients such as a pharmaceutically acceptable carrier, diluent and/or adjuvant, or combinations thereof.

The formulations may be administered by standard routes. For example, the formulations may be administered by oral, rectal, parenteral (e.g., intravenous, intraspinal, subcutaneous or intramuscular), topical, transdermal, intraperitoneal, intracranial, intracerebroventricular, intracerebral, intravaginal, or intrauterine routes.

Activin antagonists may also be incorporated, optionally along with other active agents, into biodegradable polymers allowing for sustained release, the polymers being implanted in the vicinity of where drug delivery is desired (such as at the site of a localised disease), or implanted so that the active agents are slowly released systemically. Osmotic minipumps may also be used to provide controlled delivery of high concentrations of the active agents through cannulae to the site of interest, such as directly into for example, a fibrotic growth or into the vascular supply to that growth.

### 2. Therapeutic/Pharmaceutical Compositions for Treatment of Disease

The carriers, diluents and adjuvants used in the therapeutic/pharmaceutical compositions of the invention must be "acceptable" in terms of being compatible with the other ingredients, and not being deleterious to the patient. Examples of pharmaceutically and veterinarily acceptable carriers or diluents are demineralised or distilled water; saline solution or phosphate buffered saline (PBS); gelatin; vegetable gums such as xanthan gums, alginates, agar, carrageenan, gum tragacanth or gum acacia; cellulose derivatives such as microcrystalline cellulose, methyl cellulose, ethyl cellulose, carboxymethylcellulose or hydroxypropylethylcellulose; natural or modified starches and dextrins; lactic acid-based polymers; lower alkanols, for example ethanol or iso-propanol; lower aralkanols; citrates; acetonitrile; benzyl alcohol; dimethylacetamide; dimethylformamide; monomethylacetamide; 2-pyrrolidones such as N-methylpyrrolidone; phthalates such as diethyl phthalate; polyglycolysed glycerides; lower polyalkylene glycols or lower alkylene glycols, or alkyl ethers or esters thereof, for example polyethylene glycol, polypropylene glycol, ethylene glycol, propylene glycol, 1,3-butylene glycol, propylene glycol monomethyl ether, diethylene glycol monoethyl ether and diethylene glycol monobutyl ether and polyethylene glycol fatty acid esters; fatty esters and ethers of sugars or polyhydric alcohols, and alkoxylated derivatives thereof such as alcohol ethoxylates, polyoxyethylene sorbitan- or sorbitol- fatty acid esters, polyoxyethylene fatty alcohol ethers, and ethoxylated propoxylated block copolymers; glycerol and fatty acid mono-, di- or tri- esters thereof; sorbitan esters such as sorbitan monolaurate; polysorbates; fatty acids; vegetable based oils such as peanut oil, safflower oil, olive oil, cottonseed oil, maize oil, sesame oils such as peanut oil, safflower oil, olive oil, cottonseed oil, maize oil, sesame oil, arachis oil or coconut oil; animal-derived waxes or oils, such as beeswax or lanolin, or derivatives thereof; fatty acid esters such as isopropyl palmitate, isopropyl myristate, diisobutyl adipate or ethyl oleate; fatty alcohols, such as cetostearyl alcohol; sulphated fatty alcohols; quaternary ammonium compounds; fatty sulphate esters such as dodecyl sodium sulphate; fatty aromatic sulphonates such as alkyl-benzene sulphonates or butyl-naphthalene sulphonates; alkyl naphthalene sulphonates; alkaline stearates, such as potassium or ammonium stearates; alkyl and alkaryl sulphates, such as sodium lauryl sulphate, sodium cetyl sulphate and sodium dodecylbenzenesulphonate; silicone oils, including polysiloxanes, such as methyl polysiloxane, phenyl polysiloxane and methylphenyl polysolpoxane; volatile silicones; fumed silica; colloidal silicon dioxide; magnesium aluminium silicates; mineral oils such as liquid paraffin, soft paraffin or squalane; polyvinylpyrrolidone or polymers thereof; and petroleum jelly. Typically, the carrier or carriers will form from 10% to 99.9% by weight of the compositions.

In a preferred form the pharmaceutical composition of the invention comprises as active agent an effective amount of follistatin or follistatin, together with a pharmaceutically acceptable carrier, diluent and/or adjuvant as shown in Example 1.

The pharmaceutical composition of the invention may be: in a form suitable for parenteral administration, that is, subcutaneous, intramuscular or intravenous injection; a capsule suitable for oral ingestion; an ointment, cream or lotion suitable for topical administration; in a form suitable for delivery as an eye drop; or in an aerosol form suitable for administration by inhalation, such as by intranasal inhalation or oral inhalation.

For administration as an injectable solution or suspension, non-toxic parenterally acceptable diluents or carriers can include Ringer's solution, isotonic saline, phosphate buffered saline, ethanol and 1,2 propylene glycol.

Suitable carriers, diluents, excipients and adjuvants for oral use include peanut oil, liquid paraffin, sodium carboxymethylcellulose, methylcellulose, sodium alginate, gum acacia, gum tragacanth, dextrose, sucrose, sorbitol, mannitol, gelatine and lecithin. In addition these oral formulations may contain suitable flavouring and colourings agents. When used in capsule form the capsules may be coated with compounds such as glyceryl monostearate or glyceryl distearate which delay disintegration. Alternatively, the formulations may be provided with enteric coatings such as hydroxypropylethylcellulose and acrylic and methacrylic polymers or co-polymers and/or their esters, or combinations thereof, which protect the formulation from, for example gastric juices, until at the desired locus for absorption, such as the small intestine.

Adjuvants for oral formulations may typically include one or more of emollients, emulsifiers, thickening agents, preservatives, bactericides and buffering agents.

Solid forms for oral administration may contain binders acceptable in human and veterinary pharmaceutical practice, sweeteners, colouring agents, disintegrating agents, diluents, flavourings, coating agents, preservatives, lubricants and/or time delay agents. Suitable binders include gum acacia, gelatine, corn starch or other natural or modified starches and dextrins, gum tragacanth, sodium alginate, carboxymethylcellulose or polyethylene glycol. Suitable sweeteners include sucrose, lactose, sorbitol, mannitol, xylitol, glucose, aspartame or saccharine. Many suitable colouring agents are known and will be selected according to the properties of the particular formulation, and may advantageously include natural colouring agents such as, for example, chlorophylls, carotenes, cochineal. Suitable disintegrating agents include corn starch, cellulose derivatives such as methylcellulose, polyvinylpyrrolidone, guar gum, xanthan gum, bentonite, alginic acid or agar. Suitable diluents include lactose, sorbitol, mannitol, dextrose, kaolin, cellulose, calcium carbonate, calcium silicate, citrate salts or dicalcium phosphate. Suitable flavouring agents include peppermint oil, oil of wintergreen, blackcurrant, cherry, orange, lemon or raspberry flavouring. Suitable coating agents include polymers or copolymers of acrylic acid and/or methacrylic acid and/or their esters, cellulose derivatives such as hydroxypropylethylcellulose, waxes, fatty alcohols, zein, shellac or gluten. Suitable preservatives include sodium benzoate, vitamin E, alpha-tocopherol, ascorbic acid, methyl paraben, propyl paraben or sodium bisulphite. Suitable lubricants include magnesium stearate, stearic acid, sodium oleate, sodium chloride or talc. Suitable time delay agents include glyceryl monostearate or glyceryl distearate.

Liquid forms for oral administration will typically contain, in addition to the above agents, a liquid carrier selected from, for example, water, vegetable oils such as olive oil, peanut oil, sesame oil, sunflower oil, safflower oil, arachis oil, or coconut oil, liquid paraffin, glycols such as ethylene glycol, propylene glycol, or polyethylene glycol, lower alkanols such as ethanol, propanol, or isopropanol, glycerol, fatty alcohols, triglycerides or mixtures thereof.

Suspensions for oral administration may further comprise dispersing agents and/or suspending agents selected from, for example, cellulose derivatives such as sodium carboxymethylcellulose, methylcellulose, or hydroxypropylmethylcellulose, vegetable gums such as guar gum or xanthan gum, polyvinylpyrrolidone, sodium alginate or acetyl alcohol. Suitable dispersing agents include lecithin, polyoxyethylene esters of fatty acids such as stearic acid, polyoxyethylene sorbitol mono- or di-oleate, -stearate or -laurate, polyoxyethylene sorbitan mono- or di-oleate, -stearate or -laurate and the like.

The emulsions for oral administration may further comprise one or more emulsifying agents. Suitable emulsifying agents include dispersing agents as exemplified above or other natural gums such as gum acacia or gum tragacanth.

Methods for preparing parenterally administrable compositions are apparent to those skilled in the art, and are described in more detail in, for example, Remington's Pharmaceutical Science, 15th ed., Mack Publishing Company, Easton, Pa.

The topical formulations of the present invention, comprise as active ingredient an activin antagonist, or an analogue or active fragment thereof together with one or more acceptable carriers, and optionally any other therapeutic ingredients. Formulations suitable for topical administration include liquid or semi-liquid preparations suitable for penetration through the skin to the site of where treatment is required, such as liniments, lotions, creams, ointments or pastes, and drops suitable for administration to the eye, ear or nose.

Drops according to the present invention may comprise sterile aqueous or oily solutions or suspensions. These are typically prepared by dissolving the active ingredient in an aqueous solution of a bactericidal and/or fungicidal agent and/or any other suitable preservative such as an antioxidant, and optionally including a surface active agent. The resulting solution may then be clarified by filtration, transferred to a suitable container and sterilised. Sterilisation may be achieved by, for example, autoclaving or maintaining at 90°C-100°C for half an hour, or by filtration, followed by transfer to a container by an aseptic technique. Bactericidal and fungicidal agents suitable for inclusion in the drops include, for example, phenylmercuric nitrate or acetate (0.002%), benzalkonium chloride (0.01%) and chlorhexidine acetate (0.01%). Suitable solvents for the preparation of an oily solution may include, for example, glycerol, diluted alcohol or propylene glycol.

Lotions according to the present invention include those suitable for application to the skin or eye. An eye lotion may comprise a sterile aqueous solution optionally containing a fungicide, bactericide and/or a preservative such as an antioxidant, and may be prepared by methods similar to those described above in relation to the preparation of drops. Lotions, creams or liniments for application to the skin may also include an agent to hasten drying and to cool the skin, such as an alcohol or acetone, and/or a moisturiser such as glycerol, or oil such as castor oil or arachis oil. Lotions, creams or liniments for application to the skin may also include appropriate and compatible colouring agents/dyestuffs as are well known in the art, particularly in pour-on formulations for veterinary applications so as to facilitate distinction of treated from non-treated animals.

Creams, ointments or pastes according to the present invention are semi-solid formulations of the active ingredient for external application. They may be made by mixing the active ingredient in finely-divided or powdered form, alone or in solution or suspension in an aqueous or non-aqueous fluid, with a greasy or non-greasy basis. The basis may comprise hydrocarbons such as hard, soft or liquid paraffin, glycerol or fatty acid mono-, di-, or tri- esters thereof, beeswax, a metallic soap; a mucilage; an oil of natural origin such as almond, corn, arachis, castor or olive oil; wool fat or its derivatives, or a fatty acid such as stearic or oleic acid together with an alcohol such as macrogols or glycols or ethers and/or esters thereof, for example polyethylene glycol, polypropylene glycol, ethylene glycol, propylene glycol, 1,3-butylene glycol, propylene glycol monomethyl ether, diethylene glycol monoethyl ether and diethylene glycol monobutyl ether, lower alkanols, for example ethanol or iso-propanol, lower aralkanols, or 2-pyrrolidones such as N-methylpyrrolidone.

The formulations for skin applications may also advantageously incorporate any suitable surface active agent which may aid in spreading over, or absorption through the skin, Such surfactants may be anionic, cationic, non-ionic surfactant or zwitterionic. Typically the surface active agent will be non-ionic, and more typically will be selected from fatty esters and ethers of sugars or polyhydric alcohols, and alkoxylated derivatives thereof such as alcohol ethoxylates, polyoxyethylene sorbitan- or sorbitol- fatty acid esters, polyoxyethylene fatty alcohol ethers, and ethoxylated propoxylated block copolymers. Suitable anti-foaming agents as known in the art may also be included if necessary.

Suspending and/or viscosity modifying agents such as natural gums, cellulose derivatives or inorganic materials such as silicaceous silicas, and other ingredients such as lanolin, may also be included.

Suitable preservatives for use in topical formulations according to the invention may include, for example, sodium benzoate, benzyl alcohol, vitamin E, alpha-tocopherol, ascorbic acid, 2,6-ditert-butyl-4-cresol (BHT), 2-tert-butyl-4-methoxyphenol (BHA), methyl paraben, propyl paraben or sodium bisulphite.

The pharmaceutical compositions of the invention may also be administered in the form of liposomes. Liposomes are generally derived from phospholipids or other lipid substances, and are formed by mono- or multi-lamellar hydrated liquid crystals that are dispersed in an aqueous medium. Any non-toxic, physiologically acceptable and metabolisable lipid capable of forming liposomes can be used. The formulations comprising liposomes may also contain stabilisers, preservatives, excipients and the like known in the art. For preparation of liposomes, the preferred lipids are natural and synthetic phospholipids and phosphatidyl cholines (lecithins). Methods for forming liposomes are established and published in texts such as: Prescott, Ed., Methods in Cell Biology, Volume XIV, Academic Press, New York, N.Y. (1976), p. 33 *et seq*.

### 3. Diagnosis of Disease

Screening for diseases associated with fibrosis in vertebrates using antibodies raised against activin, or follistatin, or both, can also be accomplished by any one of a number of techniques known in the art including, for example: gel diffusion precipitation reactions; immunodiffusion assays; *in situ* immunoassays; Western blots; precipitation reactions; agglutination assays; complement fixation assays; immunofluorescence assays; protein A assays; immunoelectrophoresis assays; radioimmunoassays; ELISA (enzyme-linked immunosorbent assay); sandwich immunoassays; immunoradiometric assays; receptor-binding assays; and the like.

Antibodies to activin can be raised as described previously herein, and such methods are equally applicable to the production of antibodies to follistatin.

As activin and follistatin are expressed at basal levels in normal tissue in vertebrates, detection of changes in levels of antibody-bound activin or follistatin in samples, as compared to the basal expression levels will be indicative of a disease associated with fibrosis.

Also disclosed herein is a kit for carrying out screening tests as described above which contains all the necessary reagents to carry out the test. For example, the kit may comprise the following containers:
(a) a first container containing the antibody (or fragment thereof) raised against either activin or follistatin;
(b) a second container containing a conjugate comprising a binding partner of the antibody (or fragment thereof), together with a detectable label.

It has also been found that the difference between activin and follistatin levels in tissues increases during progression of disease, detection of a change in the difference between activin and follistatin levels in a sample, as compared to the difference in a healthy, reference sample, will be indicative of a disease associated with fibrosis. A kit for carrying out screening tests as described above contains all the necessary reagents to carry out the test. For example, the kit may comprise the following containers:
(a) a first container containing at least an activin antibody (or fragment thereof), and;
(b) a second container containing at least a follistatin antibody (or fragment thereof);
(c) a third container containing a conjugate comprising a binding partner of the activin antibody (or fragment thereof), together with a detectable label, and
(d) a fourth container containing a conjugate comprising a binding partner of the follistatin antibody (or fragment thereof), together with a detectable label.

Typically, the kits described above will also comprise one or more other containers, containing for example, wash reagents, and/or other reagents capable of quantitatively detecting the presence of bound antibodies. Preferably, the detection reagents include labelled (secondary) antibodies or, where the antibody (or fragment thereof) raised against activin or follistatin is itself labelled, the compartments comprise antibody binding reagents capable of reacting with the labelled antibody (or fragment thereof) raised against activin.

In the context a compartmentalised kit includes any kit in which reagents are contained in separate containers, and may include small glass containers, plastic containers or strips of plastic or paper. Such containers allow the efficient transfer of reagents from one compartment to another compartment whilst avoiding cross-contamination of the samples and reagents, and the addition of agents or solutions of each container from one compartment to another in a quantitative fashion. Advantageously, such kits will include a container which will accept the test sample, a container which contains the antibody(s) used in the assay, containers which contain wash reagents (such as phosphate buffered saline, Tris-buffers, and like), and containers which contain the detection reagent.

### 4. Gene Therapy

The present invention also relates to compositions for use in a method of gene therapy for the treatment of disease associated with fibrosis. As described herein, gene therapy can include gene transfer and antisense biotechnological techniques.

Gene transfer can be performed by simply injecting minute amounts of DNA into the nucleus of a cell by microinjection. The introduced genes can be recognised by the cells normal mechanisms for transcription and translation, and a gene product will then be expressed.

A number of methods for introducing DNA into larger numbers of cells have also been attempted, including: transfection, which includes precipitation of target DNA with CaPO₄ which is then taken into cells by pinocytosis; electroporation, which includes exposing cells to large voltage pulses to introduce holes in the membrane through which the target DNA may pass; lipofection/liposome fusion, which includes packaging of the target DNA into lipophilic vesicles which can then fuse with a target cell; and particle bombardment whereby target DNA bound to small projectiles is forced into cells. The target DNA may also be introduced into cells by coupling the DNA to chemically modified proteins.

Typically, in a method of gene transfer according to the invention, an expression vector containing a nucleic acid molecule encoding for an activin antagonist such as follistatin or a vector comprising a nucleic acid molecule encoding the activin antagonist, is inserted into cells. The transformed cells are multiplied *in vitro* and then infused in large numbers into patients. Suitable expression vectors for delivery of these nucleic acid sequences into the targeted cell population (eg., hepatocytes, other liver cell types such as hepatic stellate cells, smooth muscle cells, lung fibroblasts, fibroblasts, kidney cells) may be derived from viruses such as retroviruses, vaccinia virus, adenovirus, adeno-associated virus, herpes viruses, several RNA viruses, or bovine papilloma virus. Recombinant viral vectors containing the target nucleic acid sequences may be prepared by any one of a number of methods which are well known to those skilled in the art. Alternatively, recombinant nucleic acid molecules encoding an activin antagonist such as follistatin can be used as naked DNA or in a reconstituted system, for example, liposomes or other lipid systems for delivery to target cells.

Typically, in a method involving antisense technology according to the invention, an expression vector containing a nucleic acid molecule encoding for a nucleic acid molecule antisense to at least a portion of a nucleotide sequence encoding activin, an activin receptor, or other activin-associated transduction pathway molecule or a vector comprising such an expression vector is inserted into cells. Suitable expression vectors may include those described above for gene transfer purposes. Typically the expression vector will be inserted in cells in a target tissue *in vivo,* and this may typically include use of a transfection vector, such as an appropriate viral vector, or use of passive uptake of the expression vector. Typically the method will include transformation of a substantial amount of the cells in the target tissue, such that expressed levels of activin, activin receptor, or other activin-associated transduction pathway molecule, are reduced compared to untransformed tissue whereby progression of the disease in the tissue is slowed, stopped or the disease recedes. Suitable viral vectors for transfection of cells with the antisense-encoding nucleic acid molecules may include attenuated versions of viruses such as adeno-associated virus, vaccinia virus, herpes viruses, several RNA viruses, retroviruses, or bovine papilloma virus.

It has also been shown that adenovirus proteins are capable of destabilising endosomes and enhancing the uptake of DNA into cells. Admixture of adenovirus to solutions containing DNA complexes, or the binding of DNA to polylysine covalently attached to adenovirus using protein crosslinking agents substantially improves the uptake and expression of the recombinant gene.

The invention will now be described in greater detail by reference to specific Examples.

### Examples

### Example 1 - Activin A and follistatin immunoreactivity indicate constitutive hepatocyte expression in normal rat liver.

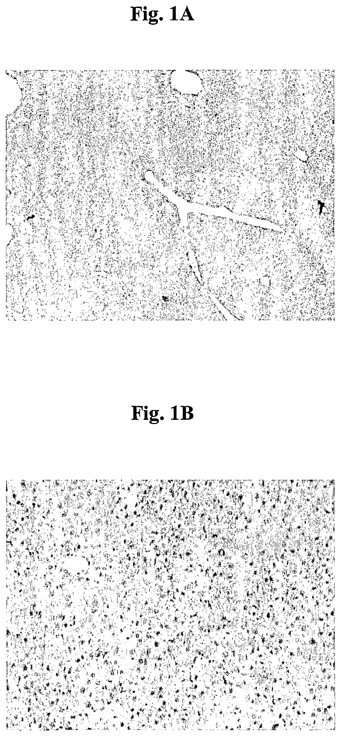

We demonstrated follistatin immunolocalisation exclusively to hepatocytes of normal liver (Figure 1).

Liver samples required for histological examination were fixed in 10% PBS buffered formalin for 24 hours at room temperature, and then washed twice in 70% alcohol and stored in 70% alcohol at room temperature until required for use. Tissue samples were then processed using the following schedule. Samples were dehydrated in sequential baths of 70% ethanol for 1 hr, 90% ethanol for 2 hrs and 100% ethanol for 2 hrs and 100% ethanol for 1 hr. Tissues were then submerged in Histosol for 1 hr 3 times in separate baths, and immersed in wax for 1 hr each in 2 separate baths. Tissues were then embedded in wax moulds and once set, stored at room temperature until required for use.

For immunohistochemistry, formalin fixed-paraffin embedded tissue blocks were sectioned at 4 µm and placed on Superfrost Plus™ slides, incubated at 60 ° C for 30 minutes and stored at room temperature until required. Slides were then deparaffinized using standard techniques and underwent microwave heat retrieval at 400 W for 10 minutes submerged in 0.1 M sodium citrate buffer (pH 7.4). Colour was developed in all staining procedures using 3'3-diaminobenzadine (DAB) unless otherwise described and slides were counter stained with hematoxylin.

To localize activin A in liver tissue, the monoclonal antibody "E₄", raised against the activin-β_{A} subunit, was applied at a final concentration of 18 µg/ml and incubated overnight at 4°C. "E₄" is the same monoclonal antibody used in the activin A ELISA and has previously been validated for both procedures (Jarred, RA, Cancilla B, Richards M, Groome NP, McNatty KP, Risbridger GP. Differential localization of inhibin subunit proteins in the ovine testis during fetal gonadal development. Endocrinology 1999; 140:979-86). Reactivity was amplified using a CSA signal amplification kit (DAKO) to manufacturer's instructions and color was developed and sections counterstained. Human prostate sections were used as a positive control, while the negative control substituted the primary antibody for an irrelevant antibody (normal mouse immunoglobulin). Since inhibin is essentially not expressed in liver, detection of the β_{A} subunit represents activin A protein and not inhibin (a heterodimer of α and β_{A} subunits).

Monoclonal antibodies against the two major isoforms of human follistatin were used to determine tissue immunoreactivity. Monoclonal antibody 17/2 (1:150) is specific against human follistatin 288, while the H10 (1:100) clone is specific for human follistatin 315 (McPherson SJ, Mellor SL, Wang H, Evans L.W, Groome NP, and Risbridger GP. Expression of activin A and follistatin core proteins by human prostate tumor cell lines. Endocrinology 1999; 140: 5303-9.). The staining procedure for each monoclonal antibody was conducted as for the activin-β_{A} subunit using cytospin preparations of the HepG2 cell line as a positive control, while an irrelevant antibody substituted for the primary antibody served as a negative control.

### Example 2 - Immunoreactivity for activin A is more intense adjacent to fibrous septa than in lobular hepatocytes but no change in follistatin expression in cirrhosis.

The expression of activin A in fibrotic and cirrhotic liver is less controversial than that of normal liver.

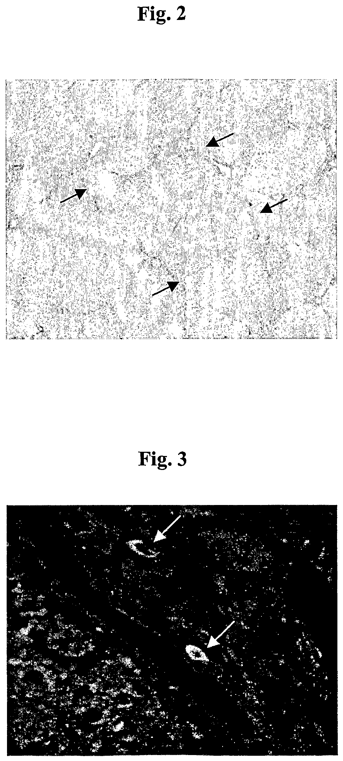

Male wistar rats weighing between 80-100 gms were housed in standard 12-hr light and dark cycles at constant temperature and humidity and allowed access to water and rat chow *ad libitum.* To establish a model of fibrosis and cirrhosis, rats were injected intraperitoneally with a 1: 1 Carbon tetrachloride/Olive oil mixture 3 times per week for 12 weeks at a dose of 0.8 ml/kg. Injections were given on three consecutive days. Control animals were injected with equal volumes of olive oil alone. In this model of fibrosis and cirrhosis we observed a change in the distribution of immunoreactive activin A from lobular hepatocytes to areas surrounding the fibrotic bands (Figure 2) and occasional co-localisation with markers of HSC's (Figure 3).

The experiment was carried out as per Example 1 and, to assess HSC's expressing activin within fibrotic septa, confocal microscopy was used. Alpha smooth muscle actin, a marker for activated hepatic stellate cells, was detected using a monoclonal antibody directed against actin filaments present in activated hepatic stellate cells. Cells expressing α-smooth muscle actin were visualised using a monoclonal mouse anti-human primary antibody applied at a final concentration of x mg/ml (1:100, DAKO corporation) for 1 hour at room temperature. This antibody has been previously validated to exhibit cross-reactivity with rat α-smooth muscle. To visualize reactivity, sections were incubated with a sheep anti-mouse-FITC conjugate (1:50, Silenus, Victoria) preabsorbed against normal rat serum for 1 hour at room temperature. To assess activin expression by intra-fibrotic HSC's, a biotinylated activin A conjugate (1:20) was applied for 1 hour and visualized with a streptavidin-Texas Red conjugate (1:50) for 30 min. Reactivity was measured under fluorescent conditions with a biorad confocal micoroscope and analysed using Biorad software.

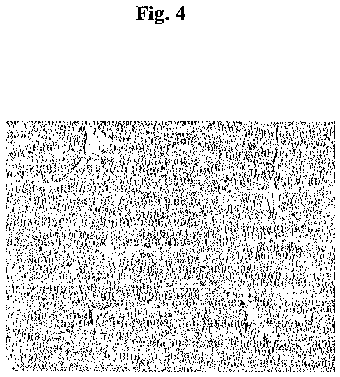

Notably, there was no discernable change in the immunoreactivity of follistatin in fibrotic and cirrhotic animals when compared to normals (Figure 4). This finding is the first description of follistatin expression in fibrotic and cirrhotic livers and would suggest that the increased activin expression adjacent to fibrous septa is unopposed by an increase in follistatin neutralising activity.

### Example 3 - Hepatic expression of activin mRNA precedes changes in follistatin mRNA during the development of hepatic fibrogenesis.

As the expression of activin and follistatin is widely distributed amongst many tissues, observations at the protein level can be confounded by accumulation of protein from extrahepatic sources.

Using real-time PCR analysis of whole liver extracts, we analysed the expression of both activin A and follistatin mRNA (Figure 5) during the model of fibrosis as described in Example 2.

Total RNA was purified using Trizol® with modifications. Briefly, after initial extraction, the supernatant was mixed with a high salt solution of 0.8M sodium citrate and 1.2M sodium chloride to allow more efficient precipitation to occur. To remove genomic DNA contamination, samples were then treated with 10U of DNase (Roche Biochemicals) at 37° for 45 mins and the reaction stopped by incubating at 95° for 3 mins. Samples were then quantitated by A₂₆₀/A₂₈₀ spectrophotometry. Two micrograms of RNA for each sample were transcribed using Superscript II® and oligo dT₁₃₋₁₅ (Gibco-BRL).

Activin β_{A} subunit mRNA analysis was performed using the Roche LightCycler (Roche) which fluorimetrically monitors the formation of PCR products in real time. This is accomplished by using the marker SYBR Green I, which in its unbound form has low fluorescence but when bound to dsDNA fluoresces strongly such that the fluorescence intensity increases in proportion to the amount of dsDNA. The log-linear portion of the PCR amplification curve is identified with the threshold or crossing point (represented in cycle number) defined as the intersection of the best-fit line through the log-linear region and the noise band. In these studies, a normal rat cDNA preparation was employed as a quality control and used in all reactions to ensure cycling conditions remained constant between experimental runs. The levels of expression of each mRNA and their estimated crossing points in each sample were determined using the LightCycler software. A ratio of specific mRNA/GAPDH amplification was then calculated. PCR reagents were purchased from Roche Biochemicals.

For PCR, 2 µl of each cDNA preparation was diluted to a final concentration of 1:400 and added to individual capillary tubes with dNTP, Mg²⁺, SYBR Green and relevant primers. Magnesium concentrations, annealing temperatures, extension times and primer specific nucleotide locations and sequences are shown in Table 1. Forty cycles of PCR were programmed to ensure that the log-linear phase was reached. At the completion of the reaction, melting curve analysis was performed to establish the specificity of the DNA products produced. PCR products were removed from the capillary tubes and visualised by gel electrophoresis to confirm the product size and integrity of the PCR reaction. In every instance, each primer set for individual animals were performed in a single PCR experiment. The intra assay variation was 4% for each primer set.

Immediately following intoxication with CCl₄ there was a significant reduction in activin A expression at 1 week which continued through to 2 weeks. Following, expression levels returned to baseline levels at 4 weeks and remained constant throughout the remainder of the model.

In contrast, follistatin expression was elevated initially relative to normal levels. Interestingly, this rise in follistatin expression correlated with peak hepatocyte proliferation as measured by PCNA expression. Following the initial rise, follistatin mRNA expression levels dropped significantly at 2 weeks, and mirrored activin expression thereafter.

It is likely that this initial response of follistatin and activin is due to the acute insult of the CCl₄ eliciting a regenerative response.

These results highlight the notion that even though activin A and follistatin levels are differentially regulated during the pathogenesis of hepatic fibrosis, this expression is inextricably linked.

### Example 4 - Activin A mRNA expression rapidly increases during the early stages of HSC activation relative to follistatin mRNA expression.

We performed real time PCR analysis on freshly isolated HSC's as they transdifferentiated *in vitro* to determine the expression pattern of activin A and follistatin in relation to other key markers of HSC proliferation and ECM production.

HSC cultures were established by sequential pronase and collagenase perfusion as previously described (Ramm GA. Isolation and culture of rat hepatic stellate cells. J Gastroenterol Hepatol (1998) 13:846-851). Briefly, cells were separated on a two-step discontinuous gradient of Nycodenz (Sigma, Sydney, Australia) and purity was assessed by characteristic vitamin A UV autofluorescence and flow cytometry. Following trypan blue exclusion to determine viability, 1 x 10⁶ cells/ml were cultured in M199 media supplemented with 10% foetal bovine and 10% normal horse serum (Trace Scientific, Victoria) in standard 5% CO₂ conditions. Media were changed after 24 hours and every 48 hours thereafter.

RNA isolation and real-time PCR were carried out as per Example 3.

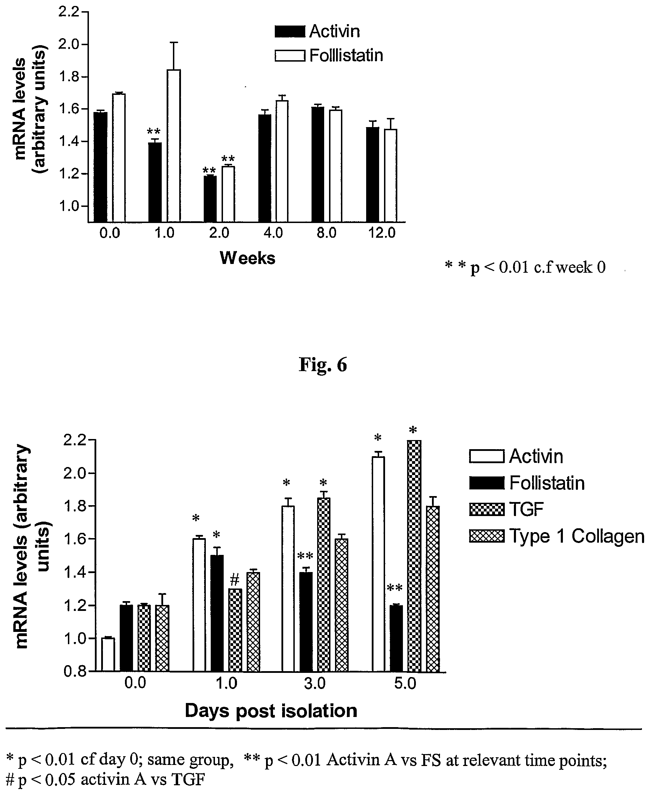

As HSC's transdifferentiated, type 1 collagen mRNA expression elevated as expected. However, our analysis revealed the induction of activin A mRNA expression very early during HSC activation which continued through to day 5 (Figure 6). Furthermore, this expression was elevated prior to the expression of TGFβ, however after day 1, TGFβ was greater than activin A.

The key finding however was the observation that follistatin mRNA expression peaked by day 1 post-isolation and remained constant until day 5 such that activin A and FS expression levels were markedly different. It has been suggested that the presence of activin A, either from an endocrine or autocrine source, is able to stimulate HSC fibronectin and collagen synthesis. These observations taken together with the findings reported in the above examples, and the knowledge that follistatin neutralizes activin A bioactivity would suggest that the addition of follistatin would be able to ameliorate HSC ECM production and therefore hepatic fibrogenesis.

### Example 5 - Isolated hepatic stellate cells produce increasing concentrations of bioactive activin A as they transform into the typical "myofibroblast-like" phenotype.

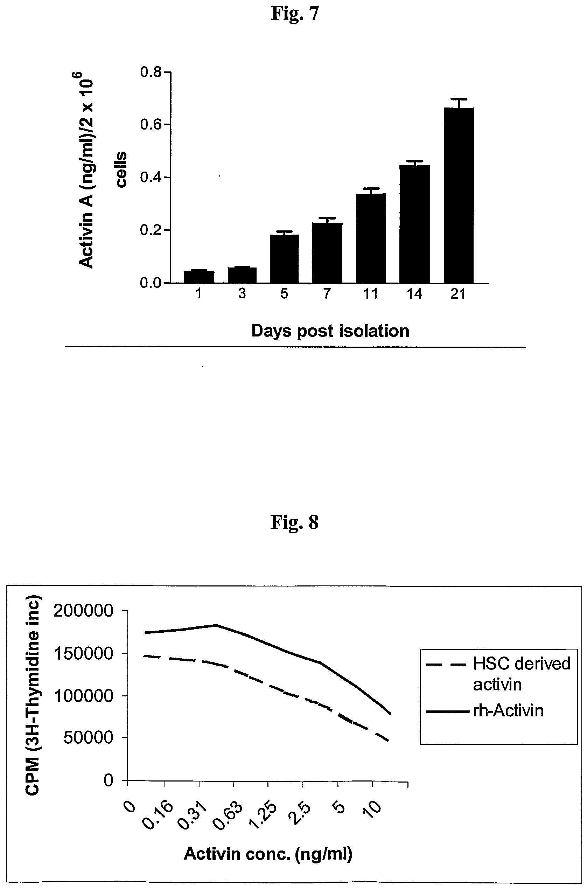

Primary cultures of HSC's were established from normal livers as per example 4 and analysis revealed the production of increasing quantities of activin A as they transformed to the activated phenotype (Figure 7).

Activin A immunoreactivity was measured by ELISA as per Knight PG, Muttukrishna S, Groome NP. Development and application of a two-site enzyme immunoassay for the determination of 'total' activin A concentrations in serum and follicular fluid was based on the method described in J Endocrinol (1996), 148:267-79.

Conditioned medium from HSC cultures were assessed for activin bioactivity using a previously validated *in vitro* bioassay (Phillips, D.J., Brauman, J.N., Mason, A.J., de Kretser, D.M. & Hedger, M.P. A sensitive and specific in vitro bioassay for activin using a mouse plasmacytoma cell line, MPC-11. J Endocrinol (1999), 162:111-115.). Addition of activin causes a dose-dependent inhibition of proliferation in mouse MPC-11 cells. These cells are refractory to TGFβ and inhibin, but the effects of activin can be blocked by addition of excess follistatin.

The addition of supernatants from HSC cultures to MPC-11 cells resulted in a dose dependant decrease in proliferation (Figure 8). This decrease in proliferation mirrored that of rh-activin A.

It was observed that the addition of culture supernatants was more potent than that observed for rh-activin A.

### Example 6 - The addition of exogenous activin A and follistatin to cultures of HSC resulted in a decrease in HSC proliferation.

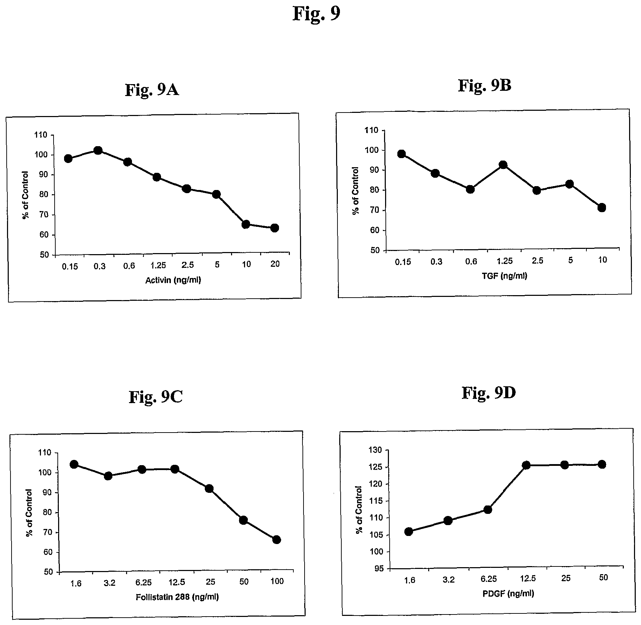

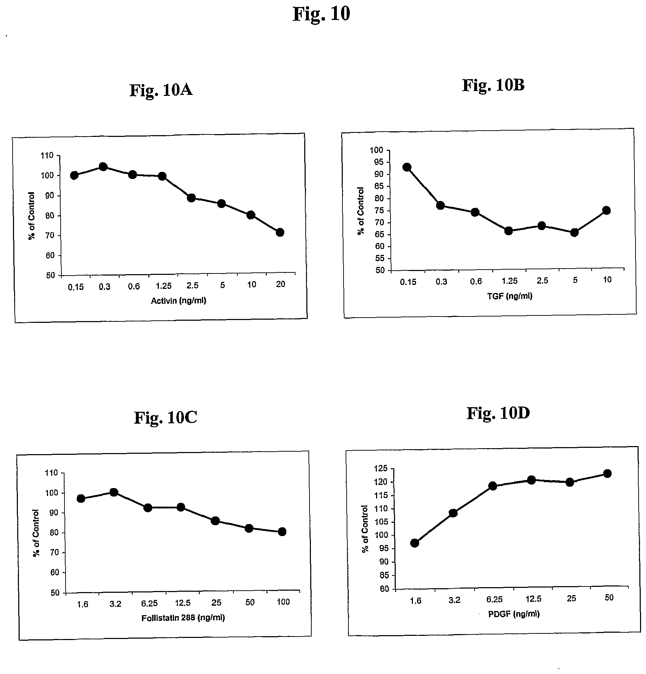

Cultures of both freshly isolated and activated HSC's, isolated as per example 4, were exposed to exogenous activin A and follistatin to determine their effects on proliferation. The addition of both activin A and follistatin resulted in dose dependent decreases in HSC proliferation in both experimental conditions (Figure 9 and 10) in a similar profile to that observed for TGFβ.

The proliferative response of HSC's and transformed HSC's (myofibroblasts) to various exogenous mediators was assessed *in vitro* by ³H-thymidine incorporation. After the optimal cellular density was determined cells were seeded onto 24 well plates at 0.5 x 10⁵ cells/well and allowed to adhere overnight at 37°C. Cell culture medium was removed and replaced with M199 media containing 0.1% BSA for 18 hours to allow cells to move into the Gₒ phase of the cell cycle. Cells were then exposed to various concentrations of Activin (0.15, 0.3, 0.6, 1.25, 2.5, 5, 10 and 20 ng/ml), TGFβ (0.15, 0.3, 0.6, 1.25, 2.5, 5, 10 ng/ml), Follistatin (1.6, 3.2, 6.25, 12.5, 25, 50 and 100 ng/ml) and PDGF-BB (1.6, 3.2, 6.25, 12.5, 25, 50 ng/ml) for 18 hrs. Cultures were then pulsed with 0.5 µCi of ³H-thymidine and incubated at 37°C for a further 16 hrs. Following, adherent and non-adherent cells were trypsinised and then harvested with a Biorad (Biorad) cell harvester onto filter paper and added to a Wallac scintillation tube. Seven hundred and fifty microlitres of scintillation fluid was added to each tube and counted for 1 min on a Wallac beta counter (Wallac).

This data suggests that follistatin may be a beneficial therapeutic agent for the treatment of hepatic fibrogenesis.

### Example 7 - Activin A has divergent effects on HSC apoptosis compared to TGFβ.

As both activin A and follistatin reduced HSC proliferation, it was hypothesized that either or both were causing HSC apoptosis.

Cultures of activated HSC's, isolated as per example 4, were exposed to varying quantities of activin A, follistatin and TGFβ and assessed by flow cytometry for the expression of annexin V; an early marker of cellular apoptosis.

Apoptosis of myofibroblast cell lines that had undergone at least 6 subcultures was measured by flow cytometry using the cell surface expression of Annexin V as a marker. Annexin V is an intracellular protein that is expressed on the cellular surface of cells undergoing apoptosis. Briefly, 2 x 10⁵ myofibroblast HSC were subcultured onto 6 well culture plates overnight. Following, cells were washed in HBSS and treated with Activin (0.1, 1, 10 and 100 ng/ml), TGFβ (1 and 5 ng/ml), Follistatin (10 and 100 ng/ml), PDGF-BB (50 ng/ml) Activin and TGFβ simultaneously (1 and 5 ng/ml respectively) and Activin and FS simultaneously (10 and 100 ng/ml respectively) for 16 hours. As a positive control, Sodium Nitroprusside (SNP) was used at a final concentration of 0.5 µM. SNP is a chemotherapeutic drug used in the treatment of various solid cancers and leukaemias. Adherent and non-adherent cells were collected after treatment with trypsin-EDTA, washed with 1 ml PBS and centrifuged at 2000 rpm for 10 min. An Annexin V-FITC specific antibody was applied for 30 min according to manufacturers instructions (Roche diagnostics) in conjunction with Propidium Iodide (PI) to distinguish between apoptotic and necrotic cells. Cells were then washed in PBS and centrifuged at 2000 rpm for 10 min and resuspended in 300 µl of PBS. The single suspension was then analysed on a MoFlo-cytomation analyzer.

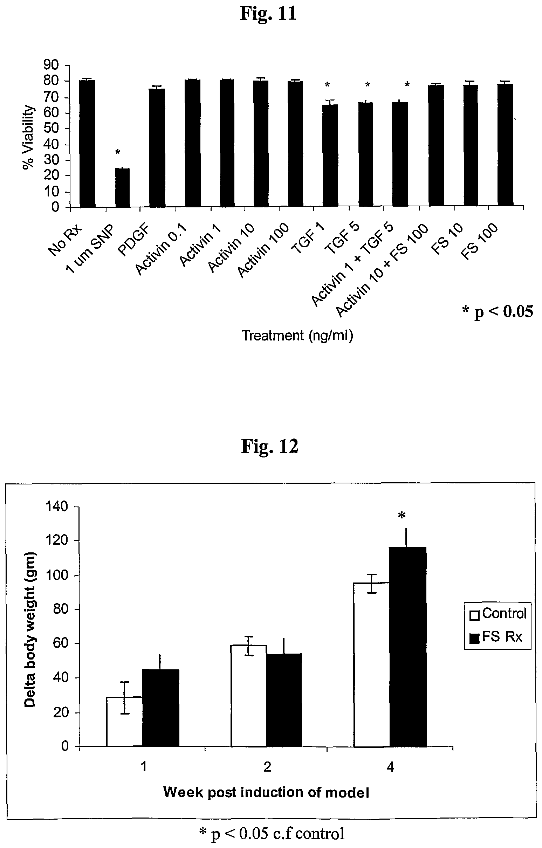

In contrast to TGFβ, the addition of activin A and follistatin did not induce HSC apoptosis (Figure 11).

This is a significant finding, as it strongly suggests a different cellular regulatory mechanism for activin A compared to TGFβ.

### Example 8 - Parenteral administration of recombinant human follistatin in a model of short term and long term liver injury.

To determine if parenteral administration of follistatin was able to reduce the progression of liver fibrosis, rats were exposed to CCl₄ either for 4 weeks (short term) or for 8 weeks (long term). Animals were then co-injected with 1 µg of follistatin 3 times a week for the first 4 weeks (short term) or from weeks 8-12 (long term) and then sacrificed. Control animals received CCl₄ for the same length of time.

Cirrhosis was induced in male Wistar rats by injecting a 1:5 vol/vol mix of carbon tetrachloride (CCl₄) and olive oil at a final concentration of 0.4mg/kg 3 times weekly for either 4 weeks (short term model) or 12 weeks (long term model). Control animals received injections of equivalent volumes of olive oil alone. Whole livers were removed at model's end for histological and mRNA and analysis as previously described above in Examples 1-3. Additionally, to determine an estimate of total liver fibrosis, hydroxyproline content was measured as previously described (Bergman, I., and R. Loxley, "Two improved and simplified methods for the spectrophotometric determination of hydroxyproline", Anal Chem (1963) 35: 1961-1965). Briefly, frozen liver samples were weighed and hydrolysed in 6N HCl acid at 110 °C for 16-18 hours in teflon coated tubes. Samples were cooled and 40 mg Dowex (Sigma)/activated charcoal was added to each sample and vortexed. The hydrolysate was filtered through 2 filter papers into a fresh tube and the samples were then brought to a pH of 7.4. The samples were then incubated with isopropanol and chloramine T for 25 minutes at 60 °C. Following cooling to room temperatures, the hydroxyproline content is estimated by reading the absorbance of the samples at a wavelength of 570 nm.

For the short term model of fibrosis, 1 µg of recombinant human FS-288 was injected intra-muscularly in the hind legs 3 times a week for the duration of the model (4 weeks). For the long term model of fibrosis and cirrhosis, animals were injected at the same dose by the same route between weeks 8 and 12 of the model.

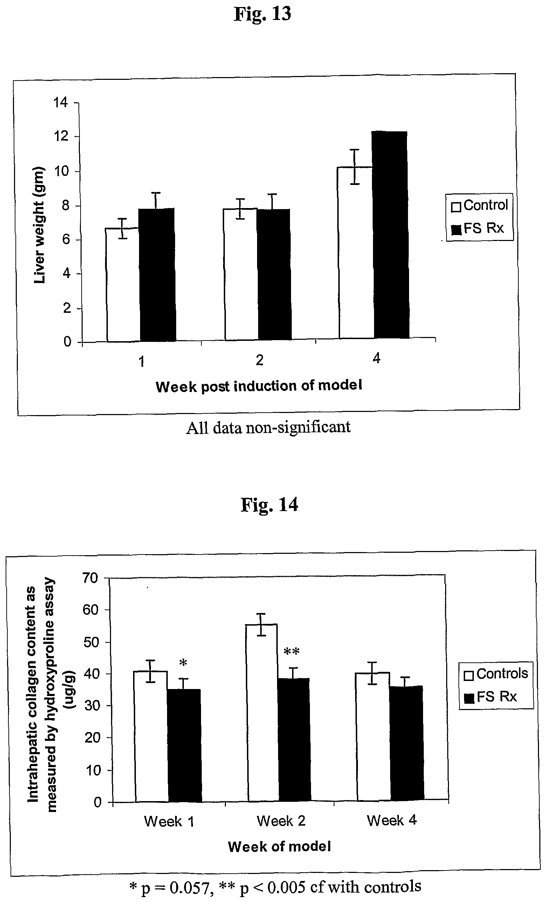

In the short term model, animals treated with follistatin had greater body weight (Figure 12) and a greater remnant liver weight (Figure 13). Furthermore, they had significantly less intrahepatic hydroxyproline content than controls at 2 weeks (Figure 14).

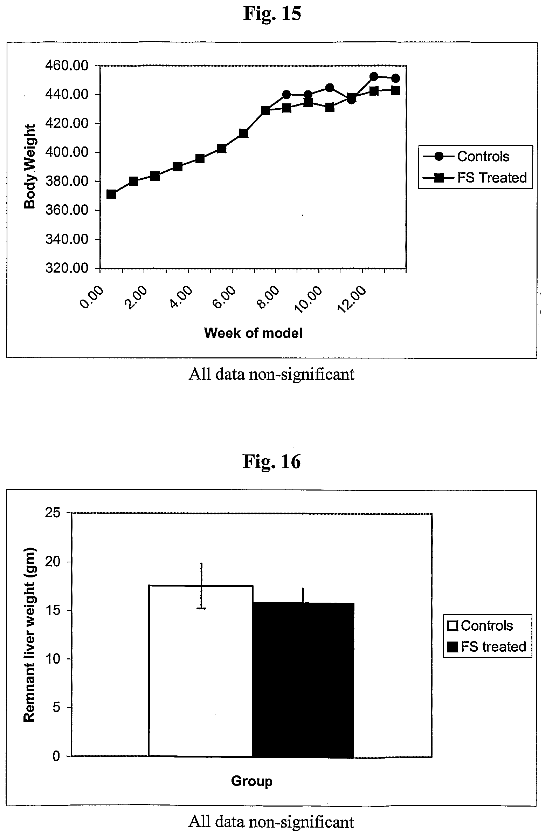

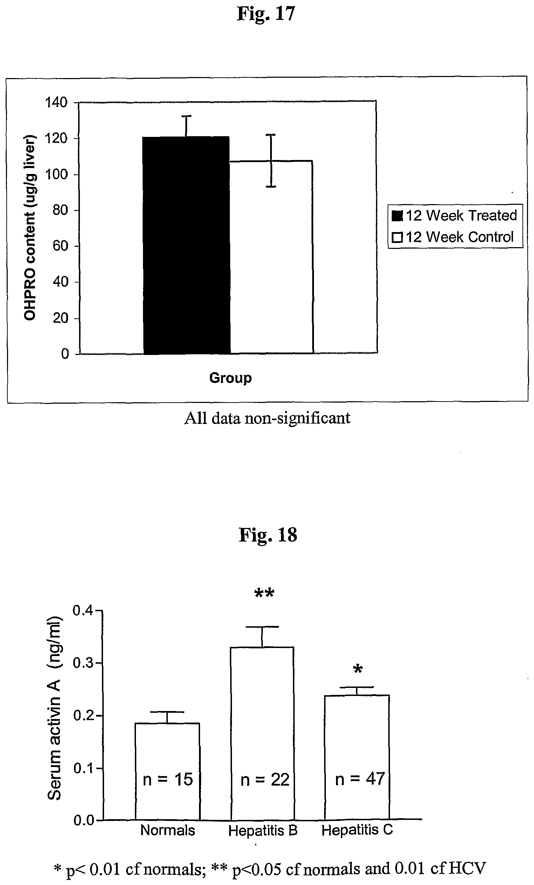

In the long term model, animals treated with follistatin showed no change in body weight (Figure 15), remnant liver weight (Figure 16) or intrahepatic hydroxyproline content (Figure 17).

From these data we conclude that the injection of follistatin may contribute to the improvement of hepatic function of remaining hepatocytes.

Furthermore, we conclude that the use of follistatin may be beneficial as a therapeutic agent to attenuate hepatic fibrogenesis in newly diagnosed patients either alone or in conjunction with current conventional therapies.

Further therapeutic applications could include: 1) aiding hepatic regeneration and collagen absorption in patients who have successfully completed conventional therapy; 2) restoring hepatic function in patients who have undergone hepatic resection and to attenuate fibrosis of regenerating liver.

### Example 9 - Studies in Humans

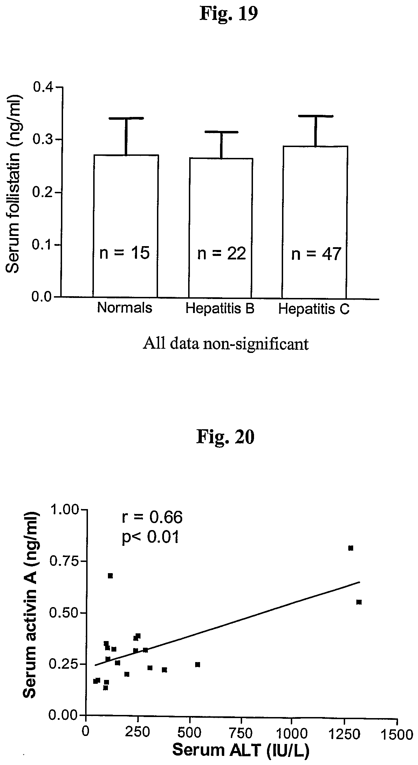

Serum activin A was demonstrated to be elevated in patients with chronic viral hepatitis compared to normal controls (Figure 18) whereas serum follistatin remained at normal levels (Figure 19), as also reported in Patella, S., et al. (2001), "Characterization of serum activin-A and follistatin and their relation to virological and histological determinants in chronic viral hepatitis", J Hepatol,, 34(4): 576-83. This study is one of the few studies that have investigated activin A in human disease and is the only study that has looked at human viral hepatitis with any detail.

Briefly, archival serum stored at -80° C was analysed from 15 normal, 22 hepatitis B and 47 hepatitis C subjects Liver function tests, virology and liver biopsy were performed during routine clinical management. Total serum activin A was measured by a two step sandwich ELISA in which human recombinant activin-A is used as the standard as described previously (Knight, P.G., Muttukrishna, S. & Groome, N.P. Development and application of a two-site enzyme immunoassay for the determination of 'total' activin-A concentrations in serum and follicular fluid. J Endocrinol 1996; 148:267-279). Total serum follistatin was quantitated using an ultra-sensitive ELISA using recombinant monoclonal antibodies as previously validated (Evans, L.W., Muttukrishna, S. & Groome, N.P. Development, validation and application of an ultra-sensitive two-site enzyme immunoassay for human follistatin. J Endocrinol 1998; 156: 275-282). The standard used was human recombinant follistatin 288 obtained from the National Hormone and Pituitary Program (Rockville, MD, USA). Immunohistochemistry for activin A subunit and follistatin was performed as detailed in Example 2.

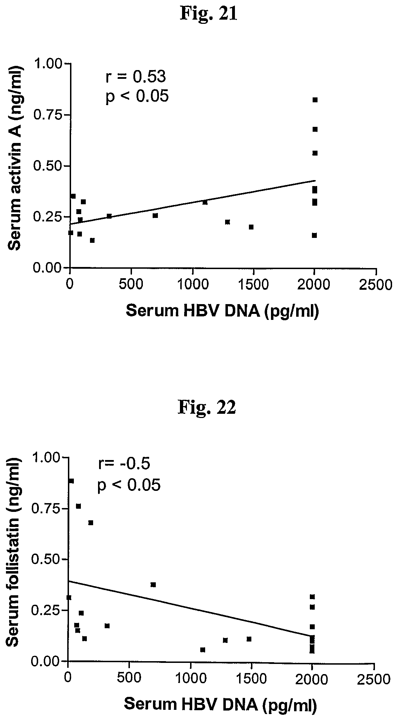

Further analysis of patients infected with HBV revealed that serum activin A significantly correlated with ALT, a marker for hepatocyte injury and intrahepatic inflammation (Figure 20). Serum activin A in patients with HBV were also observed to correlate positively with viral replication (Figure 21), whereas serum follistatin correlated negatively (Figure 22). This would suggest that the unopposed presence of activin A in this system may contribute to the pathogenesis of chronic hepatitis infection by influencing viral replication.

### Example 10 - Expression of Recombinant Human Follistatin in CHO Cells

The human follistatin 288 (FS288) gene was amplified from human ovary cDNA (Clontech) using the polymerase chain reaction. A 954 base pair fragment was generated using primers complementary to the 5' and 3' ends of the human FS288 gene. This fragment was cloned into and plasmid pGem7Zf(-) (Promega) using standard techniques. After determining that the sequence of the FS288 insert was correct the FS288 gene was subcloned into a mammalian expression vector, pDSVC, behind the SV40 early promoter. The recombinant vector was then transfected into a mammalian cell line (Chinese Hamster Ovary, CHO) by the calcium phosphate precipitation method. Transfected cells were grown in selective medium until colonies appeared. These colonies were pooled and grown in selective medium (alpha-MEM without nucleosides plus 5% dialysed FBS), with methotrexate to amplify the mouse DHFR and linked FS288 genes. Pools secreting the highest levels of FS288 were selected and dilution plated. After a few days colonies originating from single cells were visible. These colonies were transferred to separate wells in a multiwell plate. The amount of follistatin secreted from each clone was determined. One cloned cell line, cD15 which secreted about 8µg FS288/ml/24 hours, was selected and expanded to ∼1 x10⁹ cells. These cells were used to inoculate a 30 litre Braun bioreactor containing medium (alpha-MEM, glucose plus 5% FBS) and micro-carrier beads (Cytodex 2). The fermenter working volume was 20-25 litres. The cells were grown in the fermenter using a "fill-draw" method. Culture was removed periodically and replaced with fresh media and micro-carrier beads. Harvested culture medium was separated from cells by dead end filtration through 5, 1.2 and 0.2µm filters. The final filtrate was concentrated ∼5 fold by tangential flow filtration on a 10kDa cut-off ultrafiltration cartridge (AG technologies). The concentrate, containing follistatin secreted from the cells, was stored frozen at -20°C.

### Example 11 - Purification of Recombinant Human Follistatin 288

The concentrated conditioned medium containing recombinant human FS288 was thawed and subjected to heparin sepharose chromatography. Material was loaded onto a heparin sepharose column in 50mM Na phosphate buffer (Buffer A), and eluted in a gradient of buffer A and buffer B (buffer A + 2M NaCl). Fractions containing follistatin were pooled and concentrated using a YM10 membrane. The pool was loaded onto a Sephacryl S-200 HR column in PBS pH7.0. Fractions containing follistatin were pooled and again concentrated, and sterile filtered through a 0.2µm filter and stored at -20°C. The concentration of follistatin in the final pool was estimated to be ∼900µg/ml by Bradford assay and follistatin-specific ELISA (Nigel Groome). Purity was estimated to be >90% by SDS-polyacrylamide gel electrophoresis.

### Example 12 - Formulations and methods of administration

Follistatin, or an analog or active fragment thereof will be administered with or without carriers adjuvants or excipients, but will be preferably administered as a pharmaceutical formulation. For parenteral administration, the formulation will typically be an aqueous solution, and may comprise follistatin, an analogue or an active fragment thereof in an amount of from 0.001% to 5% w/v, eg., from 0.01% to 2% w/v of the formulation, although it may comprise as much as 5% w/v but preferably not in excess of 2% w/v, and more preferably from 0.01 % to 1% w/v of the formulation.

For oral administration, the formulation may comprise follistatin, an analogue or an active fragment thereof in an amount of from 0.001% to 10% by weight, eg., from 0.01 % to 5% by weight of the formulation, although it may comprise as much as 10% by weight but preferably not in excess of 5% by weight, and more preferably from 0.1% to 2% by weight of the formulation.

For topical administration, the formulation may comprise follistatin, an analogue or an active fragment thereof in an amount of from 0.005% to 5% by weight, eg., from 0.05% to 2% by weight of the formulation, although it may comprise as much as 5% by weight but preferably not in excess of 2% by weight, and more preferably from 0.1% to 1% by weight of the formulation.

In accordance with the description of the invention provided above specific preferred pharmaceutical compositions of the present invention may be prepared, and examples of which are provided below. The following specific formulations are to be construed as merely illustrative examples of formulations and not as a limitation of the scope of the present invention in any way.

### Example 12(a) - Topical Cream Composition

A typical composition for delivery as a topical cream is outlined below:

| | |
|---|---|
| Follistatin | 0.1-1.0 g |
| Methyl hydroxybenzoate | 0.2 g |
| Lanolin (Anhydrous) | 6.0 g |
| White Beeswax | 7.5 g |
| Polawax GP 200 | 25.0 g |
| Sterilised isotonic saline to | 100.0 g |

The Polawax, beeswax and lanolin are heated together at 60°C, a solution of methyl hydroxybenzoate is added and homogenisation is achieved using high speed stirring. The temperature is then allowed to fall to below 50°C. Follistatin is then added and dispersed throughout, and the composition is allowed to cool with slow speed stirring.

### Example 12(b) - Topical Lotion Composition

A typical composition for delivery as a topical lotion is outlined below:

| | |
|---|---|
| Follistatin | 0.1-1.0g |
| Methyl Hydroxybenzoate | 0.2g |
| Sorbitan Monolaurate | 1.0g |
| Polysorbate 20 | 1.0g |
| Cetostearyl Alcohol | 1.5g |
| Glycerin | 10.0g |
| Isotonic saline to | 100.00ml |

The methyl hydroxybenzoate and glycerin are dissolved in 70ml of the isotonic saline at 75°C. The sorbitan monolaurate, polysorbate 20 and cetostearyl alcohol are melted together at 75°C and added to the aqueous solution. The resulting emulsion is homogenised, allowed to cool to below 50°C with continuous stirring and follistatin is added as a suspension in the remaining water. The whole suspension is stirred until homogenised.

### Example 12(c) - Eye Drop Composition

A typical composition for delivery as an eye drop is outlined below:

| | |
|---|---|
| Follistatin | 0.01-0.1g |
| Methyl Hydroxybenzoate | 0.003g |
| Propyl Hydroxybenzoate | 0.08g |
| Purified isotonic saline to about | 100.00ml. |

The methyl and propyl hydroxybenzoates are dissolved in 70ml isotonic saline at 75°C, and the resulting solution is allowed to cool to below 50°C. Follistatin is then added, and the solution sterilised by filtration through a membrane filter (0.22 µm pore size), and aseptically packed into sterile containers.

### Example 12(d) - Composition for Inhalation Administration

For an aerosol container with a capacity of 20-30 ml: a mixture of 10-50mg of follistatin with 0.5-1.0% by weight of a lubricating agent, such as polysorbate 85 or oleic acid, was dispersed in a suitable propellant, such as freon, and put into an appropriate aerosol container for either intranasal or oral inhalation administration.

### Example 12(e) - Composition for Parenteral Administration

A pharmaceutical composition of the present invention for intramuscular injection could be prepared to contain 1mL sterile isotonic saline, and 0.5-2mg of follistatin.

Similarly, a pharmaceutical composition for intravenous infusion may comprise 250ml of sterile Ringer's solution, and 1-5mg of follistatin.

### Example 12(f) - Capsule Composition

A pharmaceutical composition of follistatin in the form of a capsule may be prepared by filling a standard two-piece hard gelatin capsule with 0.5-5.0mg of follistatin, in powdered form, 180mg of lactose, 65mg of talc and 12mg of magnesium stearate.

### Example 21(g) - Injectable Parenteral Composition

A pharmaceutical composition of this invention in a form suitable for administration by injection may be prepared by mixing 0.1% by weight of follistatin in 12% by volume propylene glycol and isotonic saline. The solution is sterilised by filtration.

### Example 12(h) - Ointment Composition

A typical composition for delivery as an ointment includes 0.1-0.5g of follistatin, together with white soft paraffin to 100.0g, dispersed to produce a smooth, homogeneous product.

### Example 12(i) - Pour-On Formulation

A typical composition for delivery as a pour-on formulation, for example for cattle, includes 0.05-0.1% by weight of follistatin, as outlined below:

| | |
|---|---|
| Follistatin | 0.05-0.10g |
| Butylated hydroxytoluene | 0.02 g |
| Brilliant Blue FCF | 0.16g |
| Xanthan gum | 0.4g |
| Benzyl alcohol | 3.0g |
| Diethyleneglycol monobutyl ether | to 100g |

If a surface active agent is desired to be included in the formulation so as to aid in spreading of the formulation over the skin and/or hair of the animal, and/or so as to aid in absorption of the formulation through the skin, a suitable surfactant, preferably an anionic surfactant such as Eco-teric T80 (polyoxyethylene (20) sorbitan monooleate) may be included in the formulation at a concentration of, for example, 20% by weight.

## Claims

1. Follistatin for use in a method of treatment of fibrosis in a vertebrate in need of said treatment, wherein said fibrosis is selected from: hyperproliferative or inflammatory fibrotic diseases; inflammatory bowel disease, ulcerative colitis and Crohn's Disease; tubular necrosis in the kidney; and liver fibrosis.

2. Follistatin for use according to claim 1, **characterised in that** the vertebrate is selected from the group consisting of human, non-human primate, mice, cattle, sheep, goats, horses, rabbits, birds, cats and dogs.

3. Follistatin for use according to claim 2, **characterised in that** the vertebrate is human.

4. Follistatin for use according to any one of claims 1 to 3, **characterised in that** the fibrosis is liver fibrosis.

5. Follistatin for use according to any one of claims 1 to 4, **characterised in that** the follistatin is a single chain protein comprising between 288 and 315 amino acids with a molecular weight of between about 30,000 and 60,000 Daltons as estimated by SDS-PAGE in the absence of reducing agents, derived from follicular fluid and able to inhibit the secretion of follicle-stimulating hormone (FSH).

6. A nucleic acid molecule encoding follistatin, or a vector comprising said nucleic acid molecule for use in a method of treatment of fibrosis in a vertebrate, wherein said fibrosis is selected from: hyperproliferative or inflammatory fibrotic diseases; inflammatory bowel disease, ulcerative colitis and Crohn's Disease; tubular necrosis in the kidney; and liver fibrosis.

7. A pharmaceutical composition for use in the treatment in a vertebrate of hyperproliferative or inflammatory fibrotic diseases; inflammatory bowel disease, ulcerative colitis; Crohn's Disease; tubular necrosis in the kidney; or liver fibrosis, wherein said composition comprises follistatin and, optionally, a pharmaceutically acceptable carrier, adjuvant and/or diluent, **characterised in that** the follistatin is a single chain protein comprising between 288 and 315 amino acids with a molecular weight of between about 30,000 and 60,000 Daltons as estimated by SDS-PAGE in the absence of reducing agents, derived from follicular fluid and able to inhibit the secretion of follicle-stimulating hormone (FSH).

8. A process for preparing the pharmaceutical composition of claim 7, **characterised in that** said process comprises homogeneously mixing follistatin with a pharmaceutically acceptable carrier, adjuvant and/or diluent.

9. The pharmaceutical composition for use of claim 7, formulated for parenteral administration.

10. Follistatin for use according to any one of claims 1 to 3, **characterised in that** the fibrosis is inflammatory bowel disease.

11. Follistatin for use according to any one of claims 1 to 3, **characterised in that** the fibrosis is tubular necrosis in the kidney.

12. Follistatin for use according to any one of claims 1 to 3, **characterised in that** the fibrosis is a hyperproliferative fibrotic disease.

13. Follistatin for use according to any one of claims 1 to 3, **characterised in that** the fibrosis is an inflammatory fibrotic disease.

## Patentansprüche

1. Follistatin zur Verwendung in einem Verfahren zur Behandlung von Fibrose bei einem Wirbeltier, das eine solche Behandlung benötigt, wobei die Fibrose ausgewählt ist aus: hyperproliferativen oder entzündlichen fibrotischen Erkrankungen; chronisch-entzündlicher Darmerkrankung; ulzerativer Colitis und Morbus Crohn; tubulärer Nekrose in der Niere; und Leberfibrose.

2. Follistatin zur Verwendung nach Anspruch 1, **dadurch gekennzeichnet, dass** das Wirbeltier ausgewählt ist aus der Gruppe bestehend aus Menschen, nichtmenschlichen Primaten, Mäusen, Rindern, Schafen, Ziegen, Pferden, Kaninchen, Vögeln, Katzen und Hunden.

3. Follistatin zur Verwendung nach Anspruch 2, **dadurch gekennzeichnet, dass** das Wirbeltier ein Mensch ist.

4. Follistatin zur Verwendung nach einem der Ansprüche 1 bis 3, **dadurch gekennzeichnet, dass** die Fibrose Leberfibrose ist.

5. Follistatin zur Verwendung nach einem der Ansprüche 1 bis 4, **dadurch gekennzeichnet, dass** das Follistatin laut Schätzung durch SDS-PAGE in Abwesenheit von Reduktionsmitteln ein zwischen 288 und 315 Aminosäuren umfassendes einkettiges Protein mit einem Molekulargewicht von zwischen etwa 30.000 und 60.000 Dalton ist, das aus follikulärer Flüssigkeit abgeleitet und in der Lage ist, die Sekretion des follikelstimulierenden Hormons (FSH) zu hemmen.

6. Nucleinsäuremolekül, das für Follistatin kodiert, oder Vektor, der das Nucleinsäuremolekül umfasst, zur Verwendung in einem Verfahren zur Behandlung von Fibrose bei einem Wirbeltier, wobei die Fibrose ausgewählt ist aus: hyperproliferativen oder entzündlichen fibrotischen Erkrankungen; chronisch-entzündlicher Darmerkrankung, ulzerativer Colitis und Morbus Crohn; tubulärer Nekrose in der Niere; und Leberfibrose.

7. Pharmazeutische Zusammensetzung zur Verwendung in der Behandlung von hyperproliferativen oder entzündlichen fibrotischen Erkrankungen; chronisch-entzündlicher Darmerkrankung, ulzerativer Colitis und Morbus Crohn; tubulärer Nekrose in der Niere; und Leberfibrose bei einem Wirbeltier, wobei die Zusammensetzung Follistatin und gegebenenfalls einen pharmazeutisch annehmbaren Träger, ein Adjuvans und/oder einen Verdünner umfasst, **dadurch gekennzeichnet, dass** das Follistatin laut Schätzung durch SDS-PAGE in Abwesenheit von Reduktionsmitteln ein zwischen 288 und 315 Aminosäuren umfassendes einkettiges Protein mit einem Molekulargewicht von zwischen etwa 30.000 und 60.000 Dalton ist, das aus follikulärer Flüssigkeit abgeleitet und in der Lage ist, die Sekretion des follikelstimulierenden Hormons (FSH) zu hemmen.

8. Verfahren zur Herstellung der pharmazeutischen Zusammensetzung nach Anspruch 7, **dadurch gekennzeichnet, dass** das Verfahren das homogene Mixen von Follistatin mit einem pharmazeutisch annehmbaren Träger, Adjuvans und/oder Verdünner umfasst.

9. Pharmazeutische Zusammensetzung zur Verwendung nach Anspruch 7, formuliert für die parenterale Verabreichung.

10. Follistatin zur Verwendung nach einem der Ansprüche 1 bis 3, **dadurch gekennzeichnet, dass** die Fibrose chronisch-entzündliche Darmerkrankung ist.

11. Follistatin zur Verwendung nach einem der Ansprüche 1 bis 3, **dadurch gekennzeichnet, dass** die Fibrose tubuläre Nekrose in der Niere ist.

12. Follistatin zur Verwendung nach einem der Ansprüche 1 bis 3, **dadurch gekennzeichnet, dass** die Fibrose eine hyperproliferative fibrotische Erkrankung ist.

13. Follistatin zur Verwendung nach einem der Ansprüche 1 bis 3, **dadurch gekennzeichnet, dass** die Fibrose eine entzündliche fibrotische Erkrankung ist.

## Revendications

1. Follistatine destinée à être utilisée dans un procédé de traitement d'une fibrose chez un vertébré ayant besoin dudit traitement, ladite fibrose étant choisie parmi : des maladies fibrotiques hyperprolifératives ou inflammatoires ; une maladie inflammatoire de l'intestin, une colite ulcéreuse et la maladie de Crohn ; une nécrose tubulaire dans le rein ; et une fibrose hépatique.

2. Follistatine destinée à être utilisée selon la revendication 1, **caractérisée en ce que** le vertébré est choisi parmi le groupe constitué d'êtres humains, primates non humains, souris, bovins, moutons, chèvres, chevaux, lapins, oiseaux, chats et chiens.

3. Follistatine destinée à être utilisée selon la revendication 2, **caractérisée en ce que** le vertébré est un être humain.

4. Follistatine destinée à être utilisée selon l'une quelconque des revendications 1 à 3, **caractérisée en ce que** la fibrose est une fibrose hépatique.

5. Follistatine destinée à être utilisée selon l'une quelconque des revendications 1 à 4, **caractérisée en ce que** la follistatine est une protéine à chaîne unique comprenant entre 288 et 315 acides aminés ayant un poids moléculaire compris entre environ 30 000 et 60 000 Daltons, comme estimé par SDS-PAGE en l'absence d'agents réducteurs, dérivés du fluide folliculaire et capables d'inhiber la sécrétion de l'hormone folliculo-stimulante (FSH).

6. Molécule d'acide nucléique codant pour la follistatine, ou vecteur comprenant ladite molécule d'acide nucléique pour une utilisation dans un procédé de traitement d'une fibrose chez un vertébré, ladite fibrose étant choisie parmi : des maladies fibrotiques hyperprolifératives ou inflammatoires ; une maladie inflammatoire de l'intestin, une colite ulcéreuse et la maladie de Crohn ; une nécrose tubulaire dans le rein ; et une fibrose hépatique.

7. Composition pharmaceutique destinée à être utilisée dans le traitement chez un vertébré de maladies fibrotiques hyperprolifératives ou inflammatoires ; d'une maladie inflammatoire de l'intestin, d'une colite ulcéreuse ; de la maladie de Crohn ; d'une nécrose tubulaire dans le rein ; ou d'une fibrose hépatique, ladite composition comprenant de la follistatine et, de manière facultative, un support, adjuvant et/ou diluant pharmaceutiquement acceptables, **caractérisée en ce que** la follistatine est une protéine à chaîne unique comprenant entre 288 et 315 acides aminés ayant un poids moléculaire compris entre environ 30 000 et 60 000 daltons, comme estimé par SDS-PAGE en l'absence d'agents réducteurs, dérivés du fluide folliculaire et capables d'inhiber la sécrétion de l'hormone folliculo-stimulante (FSH).

8. Procédé de préparation de la composition pharmaceutique selon la revendication 7, **caractérisé en ce que** ledit procédé comprend le mélange homogène de la follistatine avec un support, adjuvant et/ou diluant pharmaceutiquement acceptables.

9. Composition pharmaceutique destinée à être utilisée selon la revendication 7, formulée pour une administration parentérale.

10. Follistatine destinée à être utilisée selon l'une quelconque des revendications 1 à 3, **caractérisée en ce que** la fibrose est une maladie inflammatoire de l'intestin.

11. Follistatine destinée à être utilisée selon l'une quelconque des revendications 1 à 3, **caractérisée en ce que** la fibrose est une nécrose tubulaire dans le rein.

12. Follistatine destinée à être utilisée selon l'une quelconque des revendications 1 à 3, **caractérisée en ce que** la fibrose est une maladie fibrotique hyperproliférative.

13. Follistatine destinée à être utilisée selon l'une quelconque des revendications 1 à 3, **caractérisée en ce que** la fibrose est une maladie fibrotique inflammatoire.
